# EUROPEAN PATENT SPECIFICATION

(11) **EP 3 252 861 B1**
(45) Date of publication and mention of the grant of the patent: **26.06.2019**
(21) Application number: 16743538.7
(22) Date of filing: 29.01.2016
(51) Int. Cl.: H01M 10/0567, H01M 10/052

(54) **NON-AQUEOUS ELECTROLYTE SOLUTION, AND NON-AQUEOUS-ELECTROLYTE SECONDARY CELL USING SAME**
WASSERFREIE ELEKTROLYTLÖSUNG UND SEKUNDÄRZELLE MIT WASSERFREIEM ELEKTROLYT DAMIT
SOLUTION ÉLECTROLYTIQUE NON AQUEUSE, ET PILE RECHARGEABLE À ÉLECTROLYTE NON AQUEUX L'UTILISANT

(30) Priority: 30.01.2015 JP 2015017654
(43) Date of publication of application: 06.12.2017
(73) Proprietor: Mitsubishi Chemical Corporation, Chiyoda-ku Tokyo 100-8251 (JP)
(72) Inventor: OHASHI, Youichi, Tokyo 100-8251 (JP)
(74) Representative: Vossius & Partner Patentanwälte Rechtsanwälte mbB
(86) International application number: PCT/JP2016/052677
(87) International publication number: WO 2016/121935

(56) References cited:
- WO-A1-2015/003560
- JP-A- 2009 158 240
- JP-A- 2009 302 058
- JP-A- 2012 142 260
- US-A1- 2008 160 418
- US-A1- 2012 171 576
- US-A1- 2012 171 579

## Description

### TECHNICAL FIELD

The present invention relates to a non-aqueous electrolyte solution and a non-aqueous electrolyte solution secondary battery using the same.

### BACKGROUND ART

Along with the rapid progress of electronic devices, heightening of the capacity of secondary batteries is increasingly demanded, and non-aqueous electrolyte solution batteries such as lithium ion secondary batteries with high energy density are widely used and actively researched.

Electrolyte solutions used for non-aqueous electrolyte solution batteries are generally composed mainly of an electrolyte and a non-aqueous solvent. Electrolyte solutions used for lithium ion secondary batteries are a non-aqueous electrolyte solution in which an electrolyte such as LiPF₆ is dissolved in a mixture of a solvent having a high dielectric constant such as cyclic carbonate and a solvent having a low viscosity such as chain carbonate.

Lithium ion secondary batteries, when charged and discharged repeatedly, cause the decomposition of the electrolyte on their electrode and the deterioration of materials constituting them, leading to lowering of their capacity. In some cases, the stability of the batteries against expansion or rupture may be deteriorated.

Methods of improving the battery characteristics of lithium ion secondary batteries by using a specific non-aqueous electrolyte solution have been proposed previously. For example, Patent Document 1 reported that the use of an electrolyte solution containing maleimide or a derivative thereof suppressed a reaction of lithium metal with the electrolyte solution and improved the self-discharge ratio of a lithium battery which was stored at 60°C for 2 months. Patent Document 2 reported that the use of an electrolyte solution containing vinylene carbonate as well as a compound such as maleimide improved the charge and discharge efficiency of a silicon negative electrode. Patent Document 3 reported that use of an electrolyte solution containing a maleimide compound and 0.05 wt% to 5 wt% of a chemical species containing a hydroxyl group having a molecular weight of less than 1,000 improved the charge and discharge efficiency of a battery. Patent Document 4 and Patent Document 5 relate to a non-aqueous electrolyte including a lithium salt, an organic solvent, and an electrolyte additive. The electrolyte additive is a metastable state nitrogen-containing polymer formed by reacting Compound (A) and Compound (B). Compound (A) is a monomer having a reactive terminal functional group and Compound (B) is a heterocyclic amino aromatic derivative as an initiator. A molar ratio of Compound (A) to Compound (B) is from 10:1 to 1:10. Further, a lithium secondary battery containing the non-aqueous electrolyte is disclosed.

Patent Document 6 describes an electrolyte additive. The electrolyte additive is selected from the group consisting of maleimide derivative, bis-maleimide derivative, and combinations thereof. The patent document also discloses an electrolyte liquid and a lithium ion battery using the electrolyte additive.

### PRIOR ART DOCUMENT

### PATENT DOCUMENT

Patent Document 1: JP-A-11-219723
Patent Document 2: JP-A-2009-302058
Patent Document 3: JP-A-2012-174680
Patent Document 4: US 2012/0171579
Patent Document 5: US 2012/0171576
Patent Document 6: WO 2015/003560

### SUMMARY OF THE INVENTION

### PROBLEM TO BE SOLVED BY INVENTION

An object of the present invention is to provide a non-aqueous electrolyte solution enabling production of a non-aqueous electrolyte solution secondary battery which achieves suppressed gas generation when used in a high temperature environment and the improved residual capacity of the battery and the improved cycle characteristic thereof, and further, is excellent in discharge load characteristic (dischargeable at high rate), and to provide a non-aqueous electrolyte solution secondary battery using the electrolyte.

### MEANS FOR SOLVING THE PROBLEMS

Although the inventions described in Patent Documents 1 to 3 certainly contribute to improving some of the characteristics of the battery, the gas generation which is particularly important in battery stability has not been solved at all, and further, improvement in the cycle characteristic for a long-term period which is important in the battery characteristics has not yet been demonstrated.

The inventors of the present invention have found, after various studies to solve the above-mentioned problems, that the problems were able to be solved, and they have completed the present invention.

Thus, the gist of the present invention includes the following.
(1) A non-aqueous electrolyte solution for use in a non-aqueous electrolyte solution secondary battery including a positive electrode having a positive electrode active material capable of absorbing and releasing a metal ion, a negative electrode having a negative electrode active material capable of absorbing and releasing a metal ion, which solution contains a compound represented by the following formula (1), 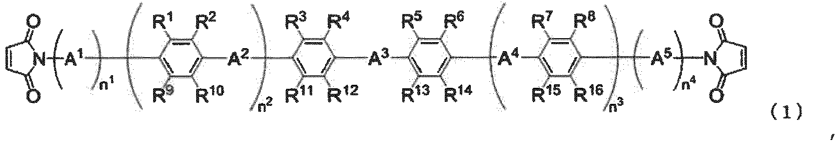
   in which each of R¹ to R¹⁶ independently represents any one of a hydrogen atom, a halogen atom, a hydrocarbon group, a group represented by -O-L¹, and a group represented by -SO₂-L²,
   L¹ and L² represent a hydrocarbon group,
   each of A¹ to A⁵ independently represents a divalent hydrocarbon group, a hetero atom, or a group having a hetero atom, and
   each of n¹ to n⁴ represents an integer of 0 or more, with the proviso that when all of n¹ to n⁴ are 0, at least one of R³ to R⁶ and R¹¹ to R¹⁴ represents a group other than a hydrogen atom, and
   wherein the compound represented by the formula (1) is a compound represented by the following
   formula (2), 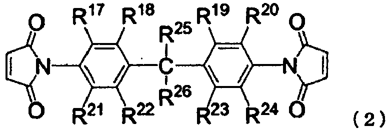
   in which each of R¹⁷ to R²⁶ independently represents any one of a hydrogen atom, a halogen atom, a hydrocarbon group, a group represented by -O-L¹, and a group represented by -SO₂-L²,
   L¹ and L² represent a hydrocarbon group, and
   at least one of R¹⁷ to R²⁴ represents a group other than a hydrogen atom.
(2) The non-aqueous electrolyte solution according to (1), wherein the content of the compound represented by the formula (1) is 0.01% by mass or more and 5% by mass or less in the non-aqueous electrolyte solution.
(3) The non-aqueous electrolyte solution according to any one of (1) to (2), wherein R¹ to R¹⁶ are a hydrogen atom or an alkyl group in the compound represented by the formula (1).
(4) The non-aqueous electrolyte solution according to any one of (1) to (3), containing a water content of 40 ppm by mass or less.
(5) The non-aqueous electrolyte solution according to any one of (1) to (4), further containing at least one of a cyclic carbonate having a carbon-carbon unsaturated bond and a cyclic carbonate having a fluorine atom.
(6) The non-aqueous electrolyte solution according to any one of (1) to (5), further containing a nitrile compound.
(7) A non-aqueous electrolyte solution secondary battery including a positive electrode having a positive electrode active material capable of absorbing and releasing a metal ion, a negative electrode having a negative electrode active material capable of absorbing and releasing a metal ion, and a non-aqueous electrolyte solution, which battery uses the non-aqueous electrolyte solution according to any one of (1) to (6).

### EFFECT OF THE INVENTION

According to the present invention, a non-aqueous electrolyte solution secondary battery can be obtained which achieves suppressed gas generation when used in a high-temperature environment, the improved residual capacity of the battery and the improved cycle characteristic thereof, and further, is excellent in discharge load characteristic (dischargeable at high rate).

### DESCRIPTION OF EMBODIMENTS

Hereinafter, modes for carrying out the present invention will be described in detail.

### [1. Non-aqueous electrolyte solution]

The non-aqueous electrolyte solution of the present invention contains an electrolyte and a non-aqueous solvent dissolving the electrolyte as in the case of typical non-aqueous electrolyte solutions, and is characterized mainly in that it further contains a bismaleimide compound having a specific structure.

### [1-1. Bismaleimide compound having specific structure]

Examples of the bismaleimide compound used in the present invention (hereinafter also referred to as the bismaleimide compound of the present invention) include the following compounds.

### 1-1-1. Compound represented by formula (1)

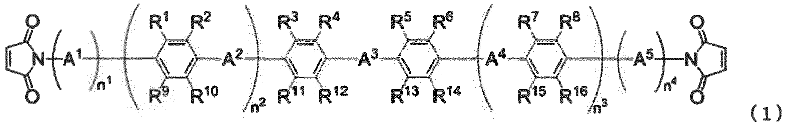

In the formula (1), each of R¹ to R¹⁶ independently represents any one of a hydrogen atom, a halogen atom, a hydrocarbon group, a group represented by -O-L¹, and a group represented by -SO₂-L².
L¹ and L² represent a hydrocarbon group.
Each of A¹ to A⁵ independently represents a divalent hydrocarbon group, a hetero atom, or a group having a hetero atom.
Each of n¹ to n⁴ each represents an integer of 0 or more, with the proviso that when all of n¹ to n⁴ are 0, at least one of R³ to R⁶ and R¹¹ to R¹⁴ represents a group other than a hydrogen atom.

Examples of the above halogen atom include fluorine, chlorine, bromine, and iodine atoms, and the fluorine atom is most preferable among them because it yields a significant effect of improving battery characteristics.

Examples of the above hydrocarbon group include alkyl, alkenyl, alkynyl, and aryl groups, and the alkyl group is most preferable among them because it leads to appropriate reactivity and low resistance.

The number of carbon atoms of the hydrocarbon group does not have any particular limitation as long as the effects of the present invention are not impaired, but it is usually 1 or more, and usually 10 or less, preferably 5 or less, more preferably 3 or less. The reason for this is that a too large number of carbon atoms leads to a too large intramolecular steric hindrance, causing difficulty in the reaction on the electrode surface.

Examples of the divalent hydrocarbon group include alkylene, alkenylene, alkynylene, and phenylene groups. Among them, the alkylene group is most preferable because it leads to appropriate reactivity and low resistance. Examples of the alkylene group include methylene, ethylene, and propylene groups, and groups which are these groups having some or all of hydrogen atoms substituted with alkyl groups, and the methylene, methylmethylene, and dimethylmethylene groups are preferable among them because they lead to low resistance.

Examples of the hetero atom include an oxygen atom and a sulfur atom, and the oxygen atom is preferable because when it is used, it leads to a preferable reactivity at the positive electrode.

Examples of the group having a hetero atom include-SO₂-, -CO₂-, -OCO₂-, -O-CH₂-, -CH₂-O-CH₂-, and -OCH₂CH₂O-groups. Among them, the -SO₂-, -CO₂-, and -OCO₂- groups are preferable because they can improve reactivity on the negative electrode.

### 1-1-2. Compound represented by formula (2)

The bismaleimide compound of the present invention is a compound represented by the following formula (2). 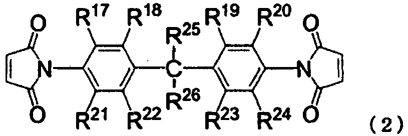 In the formula, each of R¹⁷ to R²⁶ independently represents any one of a hydrogen atom, a halogen atom, a hydrocarbon group, a group represented by -O-L¹, and a group represented by -SO₂-L². L¹ and L² are a hydrocarbon group. At least one of R¹⁷ to R²⁴ represents a group other than a hydrogen atom.

Examples of the above halogen atom include fluorine, chlorine, bromine, and iodine atoms, and the fluorine atom is most preferable among them because it yields a significant effect of improving battery characteristics.

Examples of the above hydrocarbon group include alkyl, alkenyl, alkynyl, and aryl groups, and the alkyl group is most preferable among them because it leads to appropriate reactivity and low resistance.

The number of carbon atoms of the hydrocarbon group does not have any particular limitation as long as the effects of the present invention are not impaired, but it is usually 1 or more, and usually 10 or less, preferably 5 or less, more preferably 3 or less. The reason for this is that a too large number of carbon atoms leads to a too large intramolecular steric hindrance, causing difficulty in the reaction on the electrode surface.

When all of R¹⁷ to R²⁴ are hydrogen atoms, the reactivity of the compound on the positive electrode becomes too low, reducing the effects of the present invention, and therefore, at least one of R¹⁷ to R²⁴ is desirably a group other than hydrogen atom. In order to improve the reactivity on the positive electrode, at least two of R¹⁷ to R²⁴ are preferably groups other than hydrogen atom, at least three of R¹⁷ to R²⁴ are more preferably groups other than hydrogen atom, and at least four of R¹⁷ to R²⁴ are most preferably groups other than hydrogen atom.

Specific examples of the compound represented by the formula (2) include the following compounds. The symbol Me represents methyl group. In particular, the compound 2-1, the compound 2-2, the compound 2-8, the compound 2-10, and the compound 2-11 are preferable because they have a moderate reactivity on the positive electrode surface, thereby being capable of exhibiting the effects of the present invention appropriately.

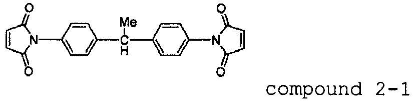

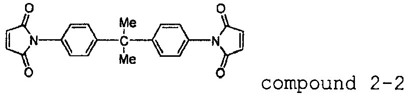

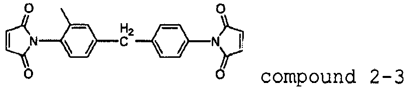

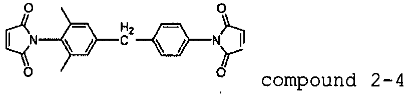

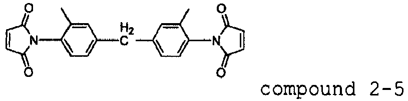

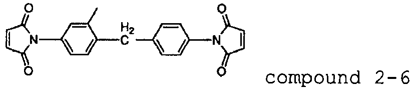

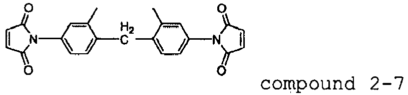

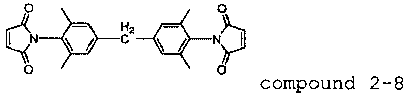

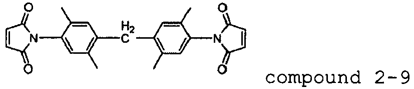

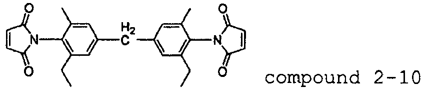

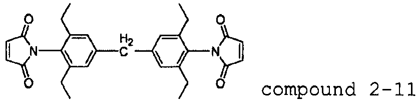

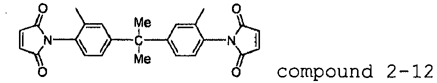

When the above compound is used, it forms a uniform coating film on the surface of the negative electrode, thereby suppressing the reduction in capacity at high temperature and being capable of reacting even on the surface of the positive electrode, thus enhancing an effect of improving the cycle characteristic. These may be used singly, or in combination of two or more kinds thereof.

When the above compound is used, it is contained preferably in an amount of 0.01% by mass or more, more preferably in an amount of 0.03% by mass or more, and most preferably in an amount of 0.05% by mass or more, in the non-aqueous electrolyte solution. It is used preferably in a content of 5% by mass or less, more preferably in a content of 3% by mass or less, particularly preferably in a content of 1.5% by mass or less, and most preferably in a content of 0.8% by mass or less. The use of the compound in the above content enables not only obtaining sufficiently an effect of improving high-temperature storage characteristics and the cycle characteristic, but also suppressing undesirable increase in resistance.

The use of the above compound enables suppressing gas generation at high temperature and the reduction of the residual capacity and further improving the cycle characteristic for a long-term period, but the reason for this is still unclear. However, the mechanism can be inferred as follows from these effects. The bismaleimide compound used in the present invention has a specific structure as described above, which is characterized in including a group having not only a benzene ring, but also a substituent or a hetero element, and the compound is thought to exhibit a lower oxidation potential relative to the positive electrode in comparison to normal bismaleimides, thus resulting in the elevation of the reactivity on the positive electrode active material. Therefore, the compound is highly capable of forming not only a coating film on the negative electrode but also a protective coating film on the positive electrode, and thus, the above-mentioned effects of characteristic improvement is presumed to be obtained.

### [1-2. Water content in electrolyte solution]

The amount of water in the non-aqueous electrolyte solution does not have any particular limitation, and it is preferably 50 ppm by mass or less, more preferably 40 ppm by mass or less, and particularly preferably 30 ppm by mass or less. Further, it is preferably 1 ppm by mass or more, more preferably 2 ppm by mass or more, and particularly preferably 3 ppm by mass or more. Within the above range, acid generation in the electrolyte solution is suppressed, and steps of production of the electrolyte solution can be relatively simplified.

### [1-3. Other compounds]

Examples of a compound which may be used in combination with the bismaleimide compound having a specific structure include a cyclic carbonate having a carbon-carbon double bond, a carbonate having a halogen atom, a nitrile compound, a compound having an S=O bond, a compound having an isocyanato group, a compound represented by a formula (X), a difluorophosphate salt, and a dicarboxylate ester.

### [1-3-1. Cyclic carbonate having carbon-carbon double bond]

Examples of the cyclic carbonate having a carbon-carbon double bond include vinylethylene carbonate compounds such as vinylene carbonate, methylvinylene carbonate, ethylvinylene carbonate, 1,2-dimethylvinylene carbonate, 1,2-diethylvinylene carbonate, fluorovinylenecarbonate, and trifluoromethylvinylene carbonate; vinylethylene carbonate compounds such as vinylethylene carbonate, 1-methyl-2-vinylethylene carbonate, 1-ethyl-2-vinylethylene carbonate, 1-n-propyl-2-vinylethylene carbonate, 1-methyl-2-vinyl ethylene carbonate, 1,1-divinylethylene carbonate and 1,2-divinylethylene carbonate; and methylene ethylene carbonate compounds such as 1,1-dimethyl-2-methylene ethylene carbonate, and 1,1-diethyl-2-methylene ethylene carbonate. These may be used singly, or in combination of two or more kinds thereof.

When the cyclic carbonate compound having a carbon-carbon double bond is contained, the content thereof in the non-aqueous electrolyte solution is usually 0.01% by mass or more, preferably 0.1% by mass or more, more preferably 0.3% by mass or more, and usually 10% by mass or less, preferably 8% by mass or less, more preferably 6% by mass or less. The content of the cyclic carbonate compound having a carbon-carbon double bond within the above range is preferable because not only the cycle characteristic is improved, but also the gas generation can be suppressed, resulting in particular improvement in battery characteristics such as reduction of internal resistance.

### [1-3-2. Carbonate having halogen atom]

The carbonate having a halogen atom does not have any particular limitation, and examples thereof include the following compounds.

In the carbonate having a halogen atom, examples of the halogen atom include fluorine, chlorine, bromine, and iodine atoms, and the fluorine and chlorine atoms are preferable, and the fluorine atom is particularly preferable among them.

Examples of the carbonate having a fluorine atom are preferably a chain carbonate having a fluorine atom and a cyclic carbonate having a fluorine atom, and examples of the chain carbonate having a fluorine atom include fluoromethylmethyl carbonate, difluoromethylmethyl carbonate, trifluoromethylmethyl carbonate, trifluoroethylmethyl carbonate, and bis(trifluoroethyl)carbonate. Among them, trifluoroethylmethyl carbonate and bis(trifluoroethyl)carbonate are preferable because they can easily form a stable coating and the stability of the compounds is also high.

Examples of the cyclic carbonate having a fluorine atom include a fluorinated product of a cyclic carbonate having an alkylene group having 2 or more and 6 or less carbon atoms and a derivative thereof, which include a fluorinated product of ethylene carbonate (hereinafter also referred to as fluorinated ethylene carbonate) and a derivative thereof. Examples of the derivative of the fluorinated product of ethylene carbonate include a fluorinated product of ethylene carbonate substituted with an alkyl group (for example, an alkyl group having 1 to 4 carbon atoms). In particular, preferable are a fluorinated ethylene carbonate having 1 or more and 8 or less fluorine atoms and a derivative thereof.

Examples of the fluorinated ethylene carbonate having 1 to 8 fluorine atoms and the derivative thereof include monofluoroethylene carbonate, 4,4-difluoroethylene carbonate, 4,5-difluoroethylene carbonate, 4-fluoro-4-methylethylene carbonate, 4,5-difluoro-4-methylethylene carbonate,4-fluoro-5-methylethylene carbonate, 4,4-difluoro-5-methylethylene carbonate, 4-(fluoromethyl)-ethylene carbonate, 4-(difluoromethyl)-ethylene carbonate, 4-(trifluoromethyl)-ethylene carbonate, 4-(fluoromethyl)-4-fluoroethylene carbonate, 4-(fluoromethyl)-5-fluoroethylene carbonate, 4-fluoro-4,5-dimethylethylene carbonate, 4,5-difluoro-4,5-dimethylethylene carbonate, and 4,4-difluoro-5,5-dimethylethylene carbonate.

In particular, monofluoroethylene carbonate, 4,4-difluoroethylene carbonate, and 4,5-difluoroethylene carbonate are preferable because they impart high ionic conductivity to the electrolyte solution and easily form a stable coating for interface protection. These may be used singly, or in combination of two or more kinds thereof.

The content of the carbonate having a halogen atom is preferably 0.01% by mass or more, more preferably 0.1% by mass or more, and most preferably 0.5% by mass or more. The carbonate is used preferably in a content of 15% by mass or less, more preferably in a content of 10% by mass or less, and most preferably in a content of 7% by mass or less. The use in the above content enables not only obtaining sufficiently the effect of improving the high-temperature storage characteristics and the cycle characteristic, but also suppressing undesirable gas generation.

### [1-3-3. Nitrile compound]

The nitrile compound does not have any particular limitation, and the examples thereof include the following compounds.

Examples of the nitrile compound include, for example,
acetonitrile, propionitrile, butyronitrile, valeronitrile, hexanenitrile, heptanenitrile, octanenitrile, nonanenitrile, decanenitrile, lauronitrile, tridecanenitrile, tetradecanenitrile, hexadecanenitrile, pentadecanenitrile, heptadecanenitrile, octadecanenitrile, nonadecanenitrile, icosanenitrile, crotononitrile, methacrylonitrile, acrylonitrile, methoxyacrylonitrile, malononitrile, succinonitrile, glutaronitrile, adiponitrile, pimelonitrile, suberonitrile, azelanitrile, sebaconitrile, undecanedinitrile, dodecanedinitrile, methylmalononitrile, ethylmalononitrile, isopropylmalononitrile, tert-butylmalononitrile, methylsuccinonitrile, 2,2-dimethylsuccinonitrile, 2,3-dimethylsuccinonitrile, 2,3,3-trimethylsuccinonitrile, 2,2,3,3-tetramethylsuccinonitrile, 2,3-diethyl-2,3-dimethylsuccinonitrile, 2,2-diethyl-3,3-dimethylsuccinonitrile, bicyclohexyl-1,1-dicarbonitrile, bicyclohexyl-2,2-dicarbonitrile, bicyclohexyl-3,3-dicarbonitrile, 2,5-dimethyl-2,5-hexanedicarbonitrile, 2,3-diisobutyl-2,3-dimethyl succinonitrile, 2,2-diisobutyl-3,3-dimethylsuccinonitrile, 2-methylglutarolnitrile, 2,3-dimethylglutaronitrile, 2,4-dimethylglutaronitrile, 2,2,3,3-tetramethylglutaronitrile, 2,2,4,4-tetramethylglutaronitrile, 2,2,3,4-tetramethylglutaronitrile, 2,3,3,4-tetramethylglutaronitrile, maleonitrile, fumaronitrile, 1,4-dicyanopentane, 2,6-dicyanoheptane, 2,7-dicyanooctane, 2,8-dicyanononane, 1,6-dicyanodecane, 1,2-dicyanobenzene, 1,3-dicyanobenzene, 1,4-dicyanobenzene, 3,3'-(ethylenedioxy)dipropionitrile, 3,3'-(ethylenedithio)dipropionitrile, and 3,9-bis(2-cyanoethyl)-2,4,8,10-tetraoxaspiro[5,5]undecane.

Among them, mononitrile, dinitrile, trinitrile, and tetranitrile are preferable from the viewpoint of handling. The reason for this is that a too large amount of nitrile causes too large toxicity of the compound. The number of carbon atoms in the mononitrile is usually 2 or more, preferably 3 or more, more preferably 4 or more, while it is usually 20 or less, preferably 18 or less, more preferably 11 or less. The number of carbon atoms in the dinitrile is usually 3 or more, preferably 4 or more, more preferably 5 or more, while it is usually 20 or less, preferably 10 or less, more preferably 8 or less. The number of carbon atoms in the trinitrile is usually 4 or more, preferably 5 or more, more preferably 6 or more, while it is usually 20 or less, preferably 18 or less, more preferably 11 or less. The number of carbon atoms in the tetranitrile is usually 5 or more, preferably 6 or more, more preferably 7 or more, while it is usually 20 or less, preferably 18 or less, more preferably 11 or less. The above ranges yield a significant effect of electrode protection, and enable suppressing increase in viscosity.

Among them, preferable are valeronitrile, octanenitrile, lauronitrile, tridecanenitrile, tetradecanenitrile, hexadecanenitrile, pentadecanenitrile, heptadecanenitrile, octadecanenitrile, nonadecanenitrile, crotononitrile, acrylonitrile, methoxyacrylonitrile, malononitrile, succinonitrile, glutaronitrile, adiponitrile, pimelonitrile, suberonitrile, azelanitrile, sebaconitrile, undecanedinitrile, dodecanedinitrile, and 3,9-bis (2-cyanoethyl)-2,4,8,10-tetraoxaspiro[5,5]undecane, and fumaronitrile, from the viewpoint of improving storage characteristics. Further, preferable are valeronitrile, octanenitrile, lauronitrile, succinonitrile, glutaronitrile, adiponitrile, pimelonitrile, suberonitrile, glutaronitrile, and 3,9-bis(2-cyanoethyl)-2,4,8,10- tetraoxaspiro[5,5]undecane, because they yield an especially excellent effect of improving storage characteristics and exhibit less deterioration caused by their side-reaction at electrodes. In general, nitrile compounds have a larger proportion of amount of the cyano group and the viscosity of the molecules increases when they have a smaller molecular weight, while they have a higher boiling point when they have a larger molecular weight. Accordingly, valeronitrile, octanenitrile, lauronitrile, succinonitrile, adiponitrile, and pimelonitrile are more preferable from the viewpoint of improving working efficiency. These may be used singly, or in combination of two or more kinds thereof.

The content of the nitrile compound is preferably 0.01% by mass or more, more preferably 0.1% by mass or more, and most preferably 0.5% by mass or more. Further, the compound is used preferably in a content of 6% by mass or less, more preferably in a content of 5% by mass or less, and most preferably in a content of 4% by mass or less. The use in the above content enables not only obtaining sufficiently the effect of improving high-temperature storage characteristics and the cycle characteristic, but also suppressing the undesirable increase in resistance.

### [1-3-4. Compound having S=O bond]

The compound having an S=O bond does not have any particular limitation, and examples thereof include the following compounds.

Examples of the compound having an S=O bond include a chain sulfonic acid ester, a cyclic sulfonic acid ester, a chain sulfuric acid ester, a cyclic sulfuric acid ester, a chain sulfurous acid ester, a cyclic sulfurous acid ester, a chain sulfone, and a cyclic sulfone. In particular, the chain sulfonic acid ester, the cyclic sulfonic acid ester, the chain sulfuric acid ester, and the cyclic sulfuric acid ester are preferably used because they yield a significant effect of improving the cycle characteristic.

Examples of the chain sulfonic acid ester include, for example, the following compounds:
a fluorosulfonic acid ester such as methyl fluorosulfonate and ethyl fluorosulfonate;
a methanesulfonic acid ester, such as methyl methanesulfonate, ethyl methanesulfonate, 2-propynyl methanesulfonate, 3-butynyl methanesulfonate, busulfan, methyl 2-(methanesulfonyloxy)propionate, ethyl 2-(methanesulfonyloxy)propionate, 2-propynyl 2-(methanesulfonyloxy)propionate, 3-butynyl 2-(methanesulfonyloxy)propionate, methyl methanesulfonyloxyacetate, ethyl methanesulfonyloxyacetate, 2-propynyl methanesulfonyloxyacetate, and 3-butynyl methanesulfonyloxyacetate;
an alkenyl sulfonic acid ester, such as methyl vinylsulfonate, ethyl vinylsulfonate, allyl vinylsulfonate, propargyl vinylsulfonate, methyl allylsulfonate, ethyl allylsulfonate, allyl allylsulfonate, propargyl allylsulfonate, and 1,2-bis(vinylsulfonyloxy)ethane; and
an alkyl disulfonic acid ester, such as methoxycarbonylmethyl methanedisulfonate, ethoxycarbonylmethyl methanedisulfonate, 1-methoxycarbonylethyl methanedisulfonate, 1-ethoxycarbonylethyl methanedisulfonate, methoxycarbonylmethyl 1,2-ethanedisulfonate, ethoxycarbonylmethyl 1,2-ethanedisulfonate, 1-methoxycarbonyl-ethyl 1,2-ethanedisulfonate, 1-ethoxycarbonylethyl 1,2-ethanedisulfonate, methoxycarbonylmethyl 1,3-propanedisulfonate, ethoxycarbonylmethyl 1,3-propanedisulfonate, 1-methoxycarbonylethyl 1,3-propanedisulfonate, 1-ethoxycarbonylethyl 1,3-propanedisulfonate, methoxycarbonylmethyl 1,3-disulfonate, ethoxycarbonylmethyl 1,3-butanedisulfonate, 1-methoxycarbonylethyl 1,3-butanedisulfonate, and 1-ethoxycarbonylethyl 1,3-butanedisulfonate.

Examples of the cyclic sulfonic acid ester include, for example, the following compounds:
a sultone compound, such as 1,3-propanesultone, 1-fluoro-1,3-propanesultone, 2-fluoro-1,3-propanesultone, 3-fluoro-1,3-propanesultone, 1-methyl-1,3-propanesultone, 2-methyl-1,3-propanesultone, 3-methyl-1,3-propanesultone, 1-propene-1,3-sultone, 2-propene-1,3-sultone, 1-fluoro-1-propene-1,3-sultone, 2-fluoro-1-propene-1,3-sultone, 3-fluoro-1-propene-1,3-sultone, 1-fluoro-2-propene-1,3-sultone, 2-fluoro-2-propene-1,3-sultone, 3-fluoro-2-propene-1,3-sultone, 1-methyl-1-propene-1,3-sultone, 2-methyl-1-propene-1,3-sultone, 3-methyl-1-propene-1,3-sultone, 1-methyl-2-propene-1,3-sultone, 2-methyl-2-propene-1,3-sultone, 3-methyl-2-propene-1,3-sultone, 1,4-butanesultone, and 1,5-pentanesultone; and
a disulfonate compound, such as methylene methanedisulfonate, and ethylene methanedisulfonate.

Examples of the chain sulfuric acid ester include the following compound:
a dialkyl sulfate compound, such as dimethyl sulfate, ethyl methyl sulfate, and diethyl sulfate.

Examples of the cyclic sulfuric acid ester include, for example, the following compound:
an alkylene sulfate compound, such as 1,2-ethylene sulfate, 1,2-propylene sulfate, 1,3-propylene sulfate, 1,2-butylene sulfate, 1,3-butylene sulfate, 1,4-butylene sulfate, 1,2-pentylene sulfate, 1,3-pentylene sulfate, 1,4-pentylene sulfate, and 1,5-pentylene sulfate.

Examples of the chain sulfurous acid ester include, for example, the following compound:
a dialkyl sulfite compound, such as dimethyl sulfite, ethylmethyl sulfite, and diethyl sulfite.

Examples of the cyclic sulfurous acid ester include, for example, the following compound:
an alkylene sulfite compound, such as 1,2-ethylene sulfite, 1,2-propylene sulfite, 1,3-propylene sulfite, 1,2-butylene sulfite, 1,3-butylene sulfite, 1,4-butylene sulfite, 1,2-pentylene sulfite, 1,3-pentylene sulfite, 1,4-pentylene sulfite, and 1,5-pentylene sulfite.

Examples of the chain sulfone include, for example, the following compound:
a dialkyl sulfone compound, such as dimethyl sulfone, and diethyl sulfone.

Examples of the cyclic sulfone include, for example, the following compound:
an alkylene sulfone compound, such as sulfolane, methylsulfolane, and 4,5-dimethylsulfolane, and alkynylene sulfone compound such sulfolene.

Among them, preferable are methyl 2-(methanesulfonyloxy)propionate, ethyl 2-(methanesulfonyloxy)propionate, 2-propynyl 2-(methanesulfonyloxy)propionate, 1-methoxycarbonylethyl propanedisulfonate, 1-ethoxycarbonylethyl propanedisulfonate, 1-methoxycarbonylethyl butanedisulfonate, 1-ethoxycarbonylethyl butanedisulfonate, 1,3-propanesultone, 1-propene-1,3-sultone, 1,4-butanesultone, 1,2-ethylenesulfate, 1,2-ethylene sulfite, methyl methanesulfonate, and ethyl methanesulfonate, from the viewpoint of improving storage characteristics, and more preferable are 1-methoxycarbonylethyl propanedisulfonate, 1-ethoxycarbonylethyl propanedisulfonate, 1-methoxycarbonylethyl butanedisulfonate, 1-ethoxycarbonylethyl butanedisulfonate, 1,3-propanesultone, 1-propene-1,3-sultone, 1,2-ethylene sulfate, and 1,2-ethylene sulfite, and further, 1,3-propanesultone and 1-propene-1,3-sultone are still more preferable. These may be used singly, or in combination of two or more kinds thereof.

The compound having an S=O bond is contained preferably in a content of 0.01% by mass or more, more preferably in a content of 0.1% by mass or more, most preferably in a content of 0.5% by mass or more. It is used preferably in a content of 5% by mass or less, more preferably in a content of 4% by mass or less, and most preferably in a content of 3% by mass or less. The use in the above content enables not only obtaining sufficiently the effect of improving the high-temperature storage characteristics and the cycle characteristic, but also suppressing the undesirable increase in resistance.

### [1-3-5. Compound having isocyanate group (N=C=O group)]

The compound having an isocyanato group (N=C=O group) does not have any particular limitation, and examples thereof include the following compounds.

Examples of the organic compound having an isocyanate group include: an organic compound having one isocyanate group, such as methyl isocyanate, ethyl isocyanate, propyl isocyanate, isopropyl isocyanate, butyl isocyanate, tert-butyl isocyanate, pentyl isocyanate,hexyl isocyanate, cyclohexyl isocyanate, vinyl isocyanate, allyl isocyanate, ethynyl isocyanate, propargyl isocyanate, phenyl isocyanate, and fluorophenyl isocyanate; and
an organic compound having two isocyanate groups, such as monomethylene diisocyanate, dimethylene diisocyanate, trimethylene diisocyanate, tetramethylene diisocyanate, pentamethylene diisocyanate, hexamethylene diisocyanate, heptamethylene diisocyanate, octamethylene diisocyanate, nonamethylene diisocyanate, decamethylene diisocyanate, dodecamethylene diisocyanate, 1,3-diisocyanatopropane, 1,4-diisocyanato-2-butene, 1,4-diisocyanato-2-fluorobutane, 1,4-diisocyanato-2,3-difluorobutane, 1,5-diisocyanato-2-pentene, 1,5-diisocyanato-2-methylpentane, 1,6-diisocyanato-2-hexene, 1,6-diisocyanato-3-hexene, 1,6-diisocyanato-diisocyanato-3-fluorohexane, 1,6-diisocyanato-3,4-difluorohexane, toluene diisocyanate, xylene diisocyanate, tolylene diisocyanate, 1,2-bis(isocyanatomethyl)cyclohexane, 1,3-bis(isocyanatomethyl)cyclohexane, 1,4-bis(isocyanatomethyl)cyclohexane, 1,2-diisocyanatocyclohexane, 1,3-diisocyanatocyclohexane, 1,4-diisocyanatocyclohexane, dicyclohexylmethane-1,1'-diisocyanate, dicyclohexylmethane-2,2'-diisocyanate, dicyclohexylmethane-3,3'-diisocyanate, dicyclohexylmethane-4, 4'-diisocyanate, bicyclo[2.2.1]heptane-2,5-diylbis(methylisocyanate), bicyclo[2.2.1] heptane-2,6-diylbis(methylisocyanate), isophorone diisocyanate, carbonyl diisocyanate, 1,4-diisocyanatobutane-1,4-dione, 1,5-diisocyanatopentane-1,5-dione, 2,2,4-trimethylhexamethylene diisocyanate, and 2,4,4-trimethylhexamethylene diisocyanate.

Among them, preferable from the viewpoint of improving storage characteristics are organic compounds having two isocyanate groups, such as monomethylene diisocyanate, dimethylene diisocyanate, trimethylene diisocyanate, tetramethylene diisocyanate, pentamethylene diisocyanate, hexamethylene diisocyanate, heptamethylene diisocyanate, octamethylene diisocyanate, nonamethylene diisocyanate, decamethylene diisocyanate, dodecamethylene diisocyanate, 1,3-bis(isocyanatomethyl)cyclohexane, dicyclohexylmethane-4,4'-diisocyanate, bicyclo[2.2.1]heptane-2,5-diylbis(methylisocyanate), bicyclo[2.2.1]heptane-2,6-diisobis(methylisocyanate), isophorone diisocyanate, 2,2,4-trimethylhexamethylene diisocyanate, and 2,4,4-trimethyl hexamethylene diisocyanate, and more preferable are hexamethylene diisocyanate, 1,3-bis(isocyanatomethyl)cyclohexane, dicyclohexylmethane-4,4'-diisocyanate, bicyclo[2.2.1]heptane-2,5-diylbis(methylisocyanate), bicyclo[2.2.1]heptane-2,6-diylbis(methylisocyanate), isophorone diisocyanate, 2,2,4-trimethylhexamethylene diisocyanate, and 2,4,4-trimethylhexamethylene diisocyanate, and still more preferable are 1,3-bis(isocyanatomethyl)cyclohexane, dicyclohexylmethane-4,4'-diisocyanate, bicyclo[2.2.1] heptane-2,5-diylbis(methylisocyanate), and bicyclo[2.2.1]heptane-2,6-diylbis(methylisocyanate). These may be used singly, or in combination of two or more kind thereof.

The content of the compound having an isocyanate group (N=C=O groups) in the non-aqueous electrolyte solution is preferably 0.01% by mass or more, more preferably 0.1% by mass or more, and most preferably 0.2% by mass or more. The compound is used preferably in a content of 5% by mass or less, more preferably in a content of 3% by mass or less, and most preferably in a content of 2% by mass or less. The use in a content within the above range enables not only obtaining sufficiently the effect of improving the high-temperature storage characteristics and the cycle characteristic, but also suppressing the undesirable increase in resistance.

### [1-3-6. Compound represented by formula (X)]

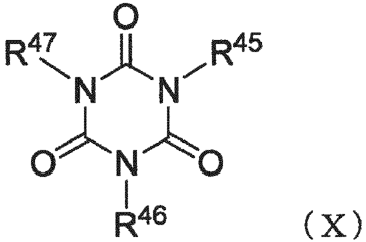

Each of R⁴⁵, R⁴⁶, and R⁴⁷ independently represents an organic group which may have a halogen atom, or a cyano, ester, or ether group.

Examples of the halogen atom represented by R⁴⁵, R⁴⁶, and R⁴⁷ include fluorine, chlorine, bromine, and iodine atoms, and the fluorine atom is most preferable among them because it exhibits a significant effect of improving battery characteristics. Examples of the organic group which may have a halogen atom, a cyano, ester, or ether group include a hydrocarbon group, such as a methyl, ethyl, propyl, isopropyl, butyl, isobutyl, t-butyl, t-amyl, vinyl, allyl, 1-propenyl, 1-butenyl, ethynyl, propynyl, phenyl, 2-tolyl, 3-tolyl, 4-tolyl, benzyl, 4-t-butylphenyl, and 4-t-amylphenyl groups, a fluorinated hydrocarbon group, such as fluoromethyl, trifluoromethyl, and trifluoroethyl groups, a cyano hydrocarbon group, such as cyanomethyl, cyanoethyl, cyanopropyl, cyanobutyl, cyanopentyl, and cyanohexyl groups, an organic group having an ester group, such as ethoxycarbonyl, ethoxycarbonylmethyl, 1-ethoxycarbonylethyl, acetoxy, acetoxymethyl, 1-acetoxyethyl, acryloyl, acryloylmethyl, and 1-acryloylethyl groups, an organic group having an ethyl group, such as methoxy, ethoxy, methoxymethyl, ethoxymethyl, methoxyethyl, and ethoxyethyl groups.

The compound represented by the formula (X) does not have any particular limitation, and examples thereof include the following compounds.

They include trivinyl isocyanurate, tri(1-propenyl)isocyanurate, triallyl isocyanurate, trimethallyl isocyanurate, methyldiallyl isocyanurate, ethyldiallyl isocyanurate, diethylallyl isocyanurate, diethylvinyl isocyanurate, tri(propargyl)isocyanurate, tris (2-acryloxymethyl)isocyanurate, tris(2-acryloxyethyl)isocyanurate, tris(2-methacryloxymethyl)isocyanurate, tris(2-methacryloxyethyl)isocyanurate, and *ε*-caprolactone-denaturated-tris-(2-acryloxyethyl)isocyanurate, and in particular, preferable are triallyl isocyanurate, trimethallyl isocyanurate, tris(2-acryloxyethyl)isocyanurate, tris(2-methacryloxyethyl)isocyanurate, and *ε*-caprolactone-denaturated-tris-(2-acryloxyethyl) isocyanurate, and particularly preferable are triallyl isocyanurate and tris(2-acryloxyethyl)isocyanurate, because they exhibit a great effect of improving the cycle characteristic. These may be used singly, or in combination of two or more kinds thereof.

The content of the compound represented by the formula (X) in the non-aqueous electrolyte solution is preferably 0.01% by mass or more, more preferably 0.1% by mass or more, most preferably 0.2% by mass or more. Further, the compound is used preferably in a content of 5% by mass or less, more preferably in a content of 3% by mass or less, and most preferably in a content of 2% by mass or less. The use in a content within the above range enables not only obtaining sufficiently the effect of improving the high-temperature storage characteristics and the cycle characteristic, but also suppressing the undesirable increase in resistance.

### [1-3-7. Difluorophosphate salt]

The difluorophosphate salt does not have particular limitation, and examples thereof include the following compounds.

For example, they include lithium difluorophosphate, sodium difluorophosphate, potassium difluorophosphate, and ammonium difluorophosphate, and lithium difluorophosphate is particularly preferable because it exhibits a significant effect of improving the cycle characteristic. These may be used singly, or in combination of two or more kinds thereof.

The content of the difluorophosphate salt in the non-aqueous electrolyte solution is preferably 0.01% by mass or more, more preferably 0.1% by mass or more, most preferably 0.2% by mass or more. Further, the compound is used preferably in a content of 3% by mass or less, more preferably in a content of 2% by mass or less, and most preferably in a content of 1.5% by mass or less. The use in a content within the above range enables not only obtaining sufficiently the effect of improving the high-temperature storage characteristics and the cycle characteristic, but also suppressing the undesirable gas generation.

### [1-3-8. Dicarboxylic acid ester]

The dicarboxylic acid ester does not have any particular limitation, and examples thereof include the following compounds:
a malonic acid ester and a derivative thereof, a succinic acid ester and a derivative thereof, an adipic acid ester and a derivative thereof, a fumaric acid ester and a derivative thereof, a maleic acid ester and a derivative thereof, a phthalic acid ester and a derivative thereof, and a terephthalic acid ester and a derivative thereof.

Examples of the malonic ester and the derivative thereof include the following compounds: dimethyl malonate, diethyl malonate, diethyl-methyl malonate, diethyl-ethyl malonate, diethyl-butyl malonate, divinyl malonate, diallyl malonate, and dipropargyl malonate.

Examples of the succinic acid ester and the derivative thereof include the following compounds: dimethyl succinate, diethyl succinate, diethyl-methyl succinate, diethyl-dimethyl succinate, diethyl-tetramethyl succinate, divinyl succinate, diallyl succinate, and dipropargyl succinate.

Examples of the adipic acid ester and the derivative thereof include the following compounds: dimethyl adipate, diethyl adipate, diethyl-methyl adipate, diethyl-dimethyl adipate, diethyl-tetramethyl adipate, divinyl adipate, diallyl adipate, and dipropargyl adipate.

Examples of the fumaric acid ester and the derivative thereof include the following compounds: dimethyl fumarate, diethyl fumarate, and diethyl-methyl fumarate.

Examples of the maleic acid ester and the derivative thereof include the following compounds: dimethyl maleate, diethyl maleate, and diethyl-methyl maleate.

Examples of the phthalic acid ester and the derivative thereof include the following compounds: dimethyl phthalate, diethyl phthalate, and di-2-ethylhexyl phthalate.

Examples of the terephthalic acid ester and the derivative thereof include the following compounds: dimethyl terephthalate, diethyl terephthalate, and di-2-ethylhexyl terephthalate.

Among them, diethyl-methyl malonate, diethyl-ethyl malonate, and diethyl-butyl malonate are particularly preferable because they exhibit a significant effect of improving the high-temperature storage characteristics. These may be used singly, or in combination of two or more kinds thereof.

The content of the dicarboxylic acid ester in the non-aqueous electrolyte solution is preferably 0.01% by mass or more, more preferably 0.1% by mass or more, most preferably 0.5% by mass or more. The compound is used preferably in a content of 5% by mass or less, more preferably in a content of 4% by mass or less, and most preferably in a content of 3% by mass or less. The use in a content within the above range enables not only obtaining sufficiently the effect of improving high-temperature storage characteristics, but also suppressing the undesirable deterioration of the cycle characteristic.

### [1-4. Electrolyte]

Any electrolyte is used, without any limitation, for the non-aqueous electrolyte solution of the present invention and any known electrolyte can be adopted as long as it can be used as an electrolyte in an objective non-aqueous electrolyte solution secondary battery. When the non-aqueous electrolyte solution of the present invention is used for lithium secondary batteries, a lithium salt is usually used as the electrolyte.

Specific examples of the electrolyte include: an inorganic lithium salt such as LiClO₄, LiAsF₆, LiPF₆, LiBF₄, LiSbF₆, LiSO₃F, and LiN(FSO₂)₂;
a fluorine-containing organic lithium salt, such as LiCF₃SO₃, LiN(FSO₂)(CF₃SO₂), LiN(CF₃SO₂)₂, LiN(C₂F₅SO₂)₂, lithium cyclic 1,3-hexafluoropropane disulfonyl imide, lithium cyclic 1,2 -tetrafluoroethane disulfonyl imide, LiN(CF₃SO₂)(C₄F₉SO₂), LiC(CF₃SO₂)₃, LiPF₄(CF₃)₂, LiPF₄(C₂F₅)₂, LiPF₄(CF₃SO₂)₂, LiPF₄(C₂F₅SO₂)₂, LiBF₂(CF₃)₂, LiBF₂(C₂F₅)₂, LiBF₂(CF₃SO₂)₂, and LiBF₂(C₂F₅SO₂)₂; and
a lithium salt complex containing dicarboxylic acid, such as lithium bis(oxalato)borate, lithium difluorooxalatoborate, lithium tris(oxalato)phosphate, lithium difluorobis(oxalato)phosphate, and lithium tetrafluoro(oxalato)phosphate.

Among them, preferable are LiPF₆, LiBF₄, LiSO₃F, LiN(FSO₂)₂, LiN(FSO₂)(CF₃SO₂), LiN(CF₃SO₂), LiN(C₂F₅SO₂)₂, lithium bis(oxalato)borate, lithium difluorooxalatoborate, lithium tris(oxalato)phosphate, lithium difluorobis(oxalato)phosphate, and lithium tetrafluoro(oxalato)phosphate, from the viewpoint of solubility and dissociation degree in a non-aqueous solvent, electric conductivity, and resultant cell characteristics, and in particular, LiPF₆ and LiBF₄ are preferable.

The electrolyte may be used singly, or in any combination of two or more kinds thereof in any combination ratio. In particular, the use of two kinds of specific inorganic lithium salts in combination, or of an inorganic lithium salt and a fluorine-containing organic lithium salt in combination is preferable because gas generation during trickle charging and deterioration after high temperature storage are suppressed. In particular, preferable is the use of LiPF₆ in combination with LiBF₄ or of an inorganic lithium salt such as LiPF₆ and LiBF₄ in combination with a fluorine-containing organic lithium salt such as LiCF₃SO₃, LiN(CF₃SO₂)₂, and LiN(C₂F₅SO₂)₂.

When LiPF₆ and LiBF₄ are used in combination, LiBF₄ is preferably contained in the electrolyte usually in a ratio thereof to the whole electrolyte which is 0.01% by mass or more and 50% by mass or less. The above ratio is preferably 0.05% by mass or more, more preferably 0.1% by mass or more, while it is preferably 20% by mass or less, more preferably 10% by mass or less, particularly preferably 5% by mass or less, most preferably 3% by mass. When the ratio is within the above range, desired effects can be easily obtained, and increase in the resistance of the electrolyte solution is suppressed owing to the low dissociation degree of LiBF₄.

On the other hand, when an inorganic lithium salt such as LiPF₆ and LiBF₄ is used in combination with an inorganic lithium salt such as LiSO₃F and LiN(FSO₂)₂, a fluorine-containing organolithium salt such as LiCF₃SO₃, LiN(CF₃SO₂)₂, LiN(C₂F₅SO₂)₂, lithium cyclic 1,3-hexafluoropropane disulfonylimide, lithium cyclic 1,2-tetrafluoroethane disulfonylimide, LiN(CF₃SO₂)(C₄F₉SO₂), LiC(CF₃SO₂)₃, LiPF₄(CF₃)₂, LiPF₄(C₂F₅)₂, LiPF₄(CF₃SO₂)₂, LiPF₄(C₂F₅SO₂)₂, LiBF₂(CF₃)₂, LiBF₂(C₂F₅)₂ , LiBF₂(CF₃SO₂)₂, and LiBF₂(C₂F₅SO₂)₂, or a lithium salt complex containing dicarboxylic acid, such as lithium bis(oxalato)borate, lithium tris(oxalato)phosphate, lithium difluorooxalatoborate, lithium tris(oxalato)phosphate, lithium difluorobis(oxalato)phosphate, and lithium tetrafluoro(oxalato)phosphate, the proportion of the inorganic lithium salt in the whole electrolyte is usually 70% by mass or more, preferably 80% by mass or more, more preferably 85% by mass or more, and usually 99% by mass or less, preferably 95% by mass or less.

The concentration of the lithium salt in the non-aqueous electrolyte solution of the present invention is arbitrary as long as the gist of the present invention is not impaired, but it is usually 0.5 mol/L or more, preferably 0.6 mol/L or more, more preferably 0.8 mol/L or more. Further, it is usually within the range of 3 mol/L or less, preferably 2 mol/L or less, more preferably 1.8 mol/L or less, still more preferably 1.6 mol/L or less. When the concentration of the lithium salt is within the above range, the electrical conductivity of the non-aqueous electrolyte solution become sufficient, decrease in the electric conductivity owing to increase in the viscosity is suppressed, and the deterioration of performance of a battery using the non-aqueous electrolyte solution of the present invention is suppressed.

### [1-5. Non-aqueous solvent]

As the non-aqueous solvent contained in the non-aqueous electrolyte solution of the present invention, a solvent can be used which is appropriately selected from conventionally known solvents as non-aqueous electrolyte solutions.

Examples of the commonly used non-aqueous solvents include a cyclic carbonate, a chain carbonate, chain and cyclic carboxylic acid esters, chain and cyclic ethers, a phosphorus-containing organic solvent, a sulfur-containing organic solvent, and an aromatic fluorine-containing solvent.

Examples of the cyclic carbonate include a cyclic carbonate such as ethylene carbonate, propylene carbonate, and butylene carbonate, and the number of carbon atoms of the cyclic carbonate is usually 3 or more and 6 or less. Among them, ethylene carbonate and propylene carbonate are preferable because they have a high dielectric constant, allowing easy dissolution of electrolytes, and thus yielding an excellent cycle characteristic when used in a non-aqueous electrolyte solution secondary battery.

Examples of the chain carbonate include a chain carbonate, such as dimethyl carbonate, ethyl methyl carbonate, diethyl carbonate, methyl-n-propyl carbonate, ethyl-n-propyl carbonate, and di-n-propyl carbonate, and the number of carbon atoms is preferably 1 or more and 5 or less, particularly preferably 1 or more and 4 or less. In particular, dimethyl carbonate, diethyl carbonate, and ethyl methyl carbonate are preferable from the viewpoint of improving battery characteristics. The examples also include a chain carbonate in which some of hydrogens in its alkyl group are substituted with fluorines. Examples of the chain carbonate substituted with fluorine include bis(fluoromethyl)carbonate, bis(difluoromethyl)carbonate, bis(trifluoromethyl)carbonate, bis(2-fluoroethyl)carbonate, bis(2,2-difluoroethyl)carbonate, bis(2,2,2-trifluoroethyl)carbonate, 2-fluoroethyl methyl carbonate, 2,2-difluoroethyl methyl carbonate, and 2,2,2-trifluoroethyl methyl carbonate.

Examples of the chain carboxylic acid ester include methyl acetate, ethyl acetate, propyl acetate, isopropyl acetate, butyl acetate, sec-butyl acetate, isobutyl acetate, t-butyl acetate, methyl propionate, ethyl propionate, propyl propionate, isopropyl propionate, methyl butyrate, ethyl butyrate, propyl butyrate, ethyl isobutyrate, ethyl isobutyrate, methyl valerate, ethyl valerate, methyl pivalate, and ethyl pivalate, and carboxylic acid esters obtained by substituting some of hydrogens in these compounds with fluorines. Examples of the chain carboxylic acid esters substituted with fluorine include methyl trifluoroacetate, ethyl trifluoroacetate, propyl trifluoroacetate, butyl trifluoroacetate, and 2,2,2-trifluoroethyl trifluoroacetate. Among them, preferable are methyl acetate, ethyl acetate, propyl acetate, butyl acetate, methyl propionate, ethyl propionate, propyl propionate, methyl butyrate, ethyl butyrate, methyl valerate, methyl isobutyrate, ethyl isobutyrate, and methyl pivalate, from the viewpoint of improving battery characteristics.

Examples of the cyclic carboxylic acid ester include γ-butyrolactone, γ-valerolactone, and the like, and cyclic carboxylic acid esters obtained by substituting some of hydrogens in these compounds with fluorines. Among them, γ-butyrolactone is more preferable.

Examples of the chain ether include dimethoxymethane, 1,1-dimethoxyethane, 1,2-dimethoxyethane, diethoxymethane, 1,1-diethoxyethane, 1,2-diethoxyethane, ethoxymethoxymethane, 1,1-ethoxymethoxyethane, and 1,2-ethoxymethoxyethane, and chain ethers obtained by substituting some of hydrogens in these compounds with fluorines. Examples of the chain ethers substituted with fluorine include bis(trifluoroethoxy)ethane, ethoxy trifluoroethoxy ethane, ethoxy-trifluoroethoxy-ethane, 1,1,1,2,2,3,4,5,5,5-decafluoro-3-methoxy-4-trifluoromethyl-pentane, 1,1,1,2,2,3,4,5,5,5-decafluoro-3-ethoxy-4-trifluoromethyl-pentane, 1,1,1,2,2,3,4,5,5,5-decafluoro-3-propoxy-4-trifluoromethyl-pentane, 1,1,2,2-tetrafluoroethyl-2,2,3,3-tetrafluoropropyl ether, and 2,2-difluoroethyl-2,2,3,3-tetrafluoropropyl ether. Among them, 1,2-dimethoxy ethane and 1,2-diethoxy ethane are more preferable.

Examples of the cyclic ether include tetrahydrofuran, 2-methyltetrahydrofuran, and 1,3-dioxane, and cyclic ethers obtained by substituting some of hydrogens in these compounds with fluorines.

Examples of the phosphorus-containing organic solvent include trimethyl phosphate, triethyl phosphate, dimethyl ethyl phosphate, diethyl methyl phosphate, ethylene methyl phosphate, ethylene ethyl phosphate, triphenyl phosphate, trimethyl phosphite, triethyl phosphite, triphenyl phosphite, trimethylphosphine oxide, triethylphosphine oxide, and triphenylphosphine oxide, and phosphorus-containing organic solvents obtained by substituting some of hydrogens in these compounds with fluorines. Examples of the phosphorus-containing organic solvent substituted with fluorine include tris(2,2,2-trifluoroethyl)phosphate, and tris(2,2,3,3,3-pentafluoropropyl)phosphate.

Examples of the sulfur-containing organic solvent include sulfolane, 2-methylsulfolane, 3-methylsulfolane, dimethyl sulfone, diethyl sulfone, ethyl methyl sulfone, methyl propyl sulfone, dimethylsulfoxide, methyl methanesulfonate, ethyl methanesulfonate, methyl ethanesulfonate, ethyl ethanesulfonate, dimethyl sulfate, diethyl sulfate, and dibutyl sulfate, and sulfur-containing organic solvents obtained by substituting some of hydrogens in these compounds with fluorines.

Examples of the aromatic fluorine-containing solvent include fluorobenzene, difluorobenzene, trifluorobenzene, tetrafluorobenzene, pentafluorobenzene, hexafluorobenzene, and benzotrifluoride.

Among the above-mentioned non-aqueous solvents, ethylene carbonate and/or propylene carbonate which is a cyclic carbonate is preferably used, and furthermore, it is more preferably used in combination with a chain carbonate from the viewpoint of compatibility between the high electrical conductivity and the low viscosity of the electrolyte solution.

The non-aqueous solvent may be used singly, or in any combination of two or more kinds thereof in any combination ratio. When two or more kinds of these solvents are used in combination, for example when cyclic carbonate and chain carbonate are used in combination, the preferable content of the chain carbonate in the non-aqueous solvent is usually 20% by volume or more, preferably 40% by volume or more, and usually 95% by volume or less, preferably 90% by volume or less. On the other hand, the preferable content of the cyclic carbonate in the non-aqueous solvent is usually 5% by volume or more, preferably 10% by volume or more, and usually 80% by volume or less, preferably 60% by volume or less. The proportion of the chain carbonate within the above range suppresses not only the viscosity increase in the non-aqueous electrolyte solution, but also the decrease in the electrical conductivity of the non-aqueous electrolyte solution caused by the decrease in the degree of dissociation of the lithium salt which is an electrolyte. In the present specification, the volume of the non-aqueous solvent is a value measured at 25°C, but for a solvent which is solid at 25°C such as ethylene carbonate, a value measured at its melting point is used as the volume.

### [1-5. Other additives]

The non-aqueous electrolyte solution of the present invention may contain various additives as long as the effects of the present invention are not significantly impaired. Any conventionally known additive can be used. The additive may be used singly, or in any combination of two or more kinds thereof in any combination ratio.

### (Overcharge inhibitor)

Specific examples of overcharge inhibitor include an aromatic compound, such as alkylbiphenyl such as 2-methylbiphenyl and 2-ethylbiphenyl, terphenyl and a partially hydrogenated product of terphenyl, cyclopentylbenzene, *cis*-1-propyl-4-phenylcyclohexane, *trans*-1-propyl-4-phenylcyclohexane, *cis*-1-butyl-4-phenylcyclohexane, *trans*-1-butyl-4-phenylcyclohexane, diphenyl ether, dibenzofuran, ethylphenyl carbonate, tris(2-t-amylphenyl)phosphate, tris(3-*t-*amylphenyl)phosphate, tris(4-*t*-amylphenyl)phosphate, tris(2-cyclohexylphenyl)phosphate, tris(3-cyclohexylphenyl)phosphate, tris(4-cyclohexylphenyl)phosphate, triphenyl phosphate, tritolyl phosphate, tri(*t*-butylphenyl)phosphate, methylphenyl carbonate, and diphenyl carbonate; a partial fluorinated compound of aromatic compounds, such as 2-fluorobiphenyl, 3-fluorobiphenyl, 4-fluorobiphenyl, 4,4'-difluorobiphenyl, 2,4-difluorobiphenyl, o-cyclohexylfluorobenzene, and *p-*cyclohexylfluorobenzene; and an fluorine-containing anisole compound, such as 2,4-difluoroanisole, 2,5-difluoroanisole, 2,6-difluoroanisole, and 3,5-difluoroanisole.

The content of these overcharge inhibitors in the non-aqueous electrolyte solution is usually 0.1% by mass or more, preferably 0.2% by mass or more, more preferably 0.3% by mass or more, still more preferably 0.5% by mass or more, and usually 5% by mass or less, preferably 3% by mass or less, more preferably 2% by mass or less. The concentration within the above range facilitates the manifestation of a desired effect of the overcharge inhibitor and suppresses the deterioration of the characteristics of the battery such as the high-temperature storage characteristics. The inclusion of the overcharge inhibitor in the non-aqueous electrolyte solution is preferable because the rupture of non-aqueous electrolyte solution secondary batteries caused by overcharge is suppressed and the stability of the non-aqueous electrolyte solution secondary batteries is improved.

Examples of other auxiliary agents include a carbonate compound, such as erythritan carbonate, spiro-bis-dimethylene carbonate, methoxyethyl-methyl carbonate, methoxyethyl-ethyl carbonate, ethoxyethyl-methyl carbonate, and ethoxyethyl-ethyl carbonate; a spiro compound, such as 2,4,8,10-tetraoxaspiro[5.5]undecane, and 3,9-divinyl-2,4,8,10-tetraoxaspiro[5.5]undecane; a nitrogen-containing compound, such as 1-methyl-2-pyrrolidinone, 1-methyl-2-piperidone, 3-methyl-2-oxazolidinone, 1,3-dimethyl-2-imidazolidinone, and N-methyl succinimide; a hydrocarbon compound, such as heptane, octane, nonane, decane, cycloheptane, methylcyclohexane, ethylcyclohexane, propylcyclohexane, *n*-butylcyclohexane, *t*-butyl cyclohexane, and dicyclohexyl; a phosphorus-containing compound, such as methyl dimethylphosphinate, ethyl dimethylphosphinate, ethyl diethylphosphinate, trimethylphosphonoformate, triethylphosphonoformate, trimethylphosphonoacetate, triethylphosphonoacetate, trimethyl-3-phosphonopropionate, and triethyl-3-phosphonopropionate; and an acid anhydride, such as succinic anhydride, methyl succinic anhydride, 4,4-dimethyl succinic anhydride, 4,5-dimethyl succinic anhydride, maleic anhydride, citraconic anhydride, dimethyl maleic anhydride, phenyl maleic anhydride, diphenyl maleic anhydride, phthalic anhydride, cyclohexane 1,2-dicarboxylic anhydride, acetic anhydride, propionic anhydride, acrylic anhydride, and methacrylic anhydride. Among them, succinic anhydride, maleic anhydride, and methacrylic anhydride are preferable from the viewpoint of improving the cycle characteristic and improvement in the high temperature storage characteristics. These may be used singly, or in combination of two or more kinds thereof.

The content of these auxiliary agents in the non-aqueous electrolyte solution does not have any particular limitation, and is usually 0.01% by mass or more, preferably 0.1% by mass or more, more preferably 0.2% by mass or more, and usually 8% by mass or less, preferably 5% by mass or less, more preferably 3% by mass or less, still more preferably 2% by mass or less. The addition of these auxiliary agents is preferable from the viewpoint of improving capacity retention characteristics after storage at high temperature and the cycle characteristic. The concentration within the above range facilitates the manifestation of the effect of the auxiliary agents and suppresses the deterioration of battery characteristics such as high rate discharge characteristics.

### [2. Negative electrode]

A negative electrode active material used for negative electrodes will be described below. The negative electrode active material does not have any particular limitation as long as it can store and release lithium ions electrochemically. Specific examples thereof include a carbonaceous material, an alloy material, and a lithium-containing metal oxide composite material. These may be used singly, or in any combination of two or more kinds thereof.

### <Negative electrode active material>

Examples of the negative electrode active material include carbonaceous materials, alloy materials, and lithium-containing metal oxide composite materials.

Examples of the carbonaceous material include (1) natural graphites, (2) artificial graphites, (3) amorphous carbons, (4) carbon-coated graphites, (5) graphite-coated graphites, and (6) resin-coated graphites.
(1) Examples of the natural graphites include scaly graphite, flaky graphite, soil graphite and/or graphite particles obtained through a treatment such as spheroidization and densification of these graphites. Among them, spherical or ellipsoidal graphite subjected to the spheroidization treatment is particularly preferable from the viewpoint of the packing density of the particles and charging/discharging rate characteristics.

Examples as an apparatus to be used in the spheronization treatment include, for example, an apparatus which repeatedly applies, to particles, a mechanical action(s), mainly impact force such as compression, friction, and shearing force, including the interaction of the particles. Specifically, preferable is an apparatus which has, inside its casing, a rotor provided with a large number of blades, which rotor rotates at a high speed, thereby applying a mechanical action, such as impact compression, friction, and shearing force to the carbon material introduced into the machine, to perform the shperonization treatment. Further, the examples are preferably machines which have a mechanism repeatedly adding a mechanical action by circulating the carbon material.
(2) Examples of the artificial graphites include a graphite produced by graphitizing an organic compound, such as coal tar pitch, coal-based heavy oil, atmospheric residual oil, petroleum-based heavy oil, an aromatic hydrocarbon, a nitrogen-containing cyclic compound, a sulfur-containing cyclic compound, polyphenylene, polyvinyl chloride, polyvinyl alcohol, polyacrylonitrile, polyvinyl butyral, natural polymer, polyphenylene sulfide, polyphenylene oxide, furfuryl alcohol resin, phenolformaldehyde resin, and imide resin, at a temperature in the range from usually 2,500°C or higher to usually 3,200°C or lower, and further as necessary, pulverizing and/or classifying the resultant graphite. In this production, a compound such as a silicon-containing compound and a boron-containing compound can be used as a graphitizing catalyst. Further, the examples also include an artificial graphite obtained by graphitizing mesocarbon microbeads separated during the heat-treatment of pitch. Still further, the examples include an artificial graphite of granulated particles composed of primary particles. For example, they include graphite particles composed of a plurality of flattened particles aggregated or bound so that the orientation surfaces thereof are not parallel with each other, wherein the graphite particles are obtained by mixing, graphitizing, and as necessary, pulverizing a graphitizable carbonaceous material powder such as coke and mesocarbon microbeads, a graphitizable binder such as tar and pitch, and a graphitization catalyst.
(3) Examples of the amorphous carbons include amorphous carbon particles obtained through heat-treatment at least once at a temperature within the non-graphitization temperature range (from 400 to 2,200°C) by using a graphitizable carbon precursor such as tar and pitch as a raw material, and amorphous carbon particles obtained through heat-treatment by using a non-graphitizable carbon precursor such as resin as a raw material.
(4) Examples of the carbon-coated graphites include a carbon graphite composite composed of natural graphite and/or artificial graphite as a core graphite and amorphous carbon coating the core graphite, obtained from natural graphite and/or artificial graphite, mixed with a carbon precursor which is an organic compound, such as tar, pitch, and resin, and then heat-treated at 400 to 2,300°C at least once. The composite may take a form in which the entire surface or a part of the surface thereof is coated, or may be a composite of a plurality of primary particles bound to each other by a binder that is carbon originating from the carbon precursor. The graphite composite can also be obtained from natural graphite and/or artificial graphite, wherein the graphite is reacted at a high temperature with a hydrocarbon-based gas, such as benzene, toluene, methane, propane, and an aromatic volatile component, for carbon to be deposited (CVD) on the surface of the graphite.
(5) Examples of the graphite-coated graphites include a graphite-coated graphite composed of natural graphite and/or artificial graphite as a core graphite and a graphitized material coating the whole or a part of the surface of the core graphite, obtained from natural graphite and/or artificial graphite, mixed with a carbon precursor that is a graphitizable organic compound, such as tar, pitch, and resin, and then heat-treated at least once at a temperature within the range of about 2,400 to 3,200°C.
(6) Examples of the resin-coated graphites include a resin-coated graphite composed of natural graphite and/or artificial graphite as a core graphite and a resin coating the surface of the core graphite, obtained by mixing a natural graphite and/or artificial graphite with resin among others, and then drying the mixture at a temperature of less than 400°C.

The carbonaceous materials (1) to (6) may be used singly or in any combination of two or more kinds thereof in any combination ratio.

Examples of an organic compounds for the above descriptions (2) to (5), such as tar, pitch, and resin include a carbonizable organic compound selected from the group consisting of coal-based heavy oil, DC heavy oil, decomposed petroleum-based heavy oil, aromatic hydrocarbon, an N-ring compound, an S-ring compound, polyphenylene, organic synthetic polymers, natural polymer, thermoplastic resins, and thermosetting resins. Furthermore, the raw organic compound may be used in a state dissolved into an organic solvent having a low molecular weight, in order to adjust the viscosity of the compound during its mixing.

The natural graphite and/or the artificial graphite as a raw material of the core graphite is preferably natural graphite subjected to the spheroidization treatment.

The alloy material for the negative electrode active material may be, without any particular limitation, any one of single lithium, a single metal and an alloy that compose a lithium alloy, or an oxide thereof, a carbide, a nitride, a silicide, a sulfide, and a phosphide. The single metal and the alloy that compose a lithium alloy is preferably a material containing a metal/metalloid element of groups 13 and 14 (that is, elements other than carbon), and more preferably a single metal of aluminum, silicon, and tin, and an alloy or a compound containing these atoms. These may be used singly, or in any combination of two or more kinds thereof in any combination ratio.

### <Physical characteristics of carbonaceous material>

When a carbonaceous material is used as the negative electrode active material, it preferably has the following physical characteristics.

### (X-ray parameter)

The d value (interlayer distance) of the lattice plane (002 plane) of the carbonaceous material determined by X-ray diffraction according to a method of Japan Society for the Promotion of Science is usually 0.335 nm or more, and usually 0.360 nm or less, preferably 0.350 nm or less, more preferably 0.345 nm or less. In addition, the crystallite size (Lc) of the carbonaceous material determined by X-ray diffraction according to the method of Japan Society for the Promotion of Science is preferably 1.0 nm or more, and in particular, more preferably 1.5 nm or more.

### (Volume-based average particle diameter)

The volume-based average particle diameter of the carbonaceous material is an average particle diameter (median diameter) based on a volume determined by a laser diffraction/scattering method, and is usually 1 µm or more, preferably 3 µm or more, more preferably 5 µm or more, particularly preferably 7 µm, and usually 100 µm or less, preferably 50 µm or less, more preferably 40 µm or less, still more preferably 30 µm or less, particularly preferably 25 µm or less.

When the volume-based average particle diameter falls below the above range, irreversible capacity loss increases, possibly leading to the loss of initial battery capacity. In addition, when it exceeds the above range, a non-uniform coated surface tends to be formed during the preparation of the electrode by coating, possibly leading to an undesirable result for battery manufacturing processes.

The volume-based average particle diameter is measured for carbon powder dispersed in 0.2% by mass of an aqueous solution (about 10 mL) of polyoxyethylene (20) sorbitan monolaurate that is a surfactant, by using a laser diffraction/scattering particle size analyzer (for example, LA-700 manufactured by HORIBA, Ltd.). The median diameter obtained by the measurement is defined as the volume-based average particle diameter of the carbonaceous material of the present invention.

### (Raman R value)

The Raman R value of the carbonaceous material is a value measured by laser Raman spectroscopy and is usually 0.01 or more, preferably 0.03 or more, more preferably 0.1 or more, and usually 1.5 or less, preferably 1.2 or less, more preferably 1 or less, particularly preferably 0.5 or less.

If the Raman R value falls below the above range, the crystallinity of the particle surface may become too high, leading to decrease in interlayer sites to be occupied by Li in response to charge and discharge. In other words, charging acceptability may decrease. In addition, when the negative electrode is densified by pressing after coating of the current collector, the crystals tend to be oriented in a direction parallel to the electrode plate, possibly leading to the deterioration of the load characteristic.

On the other hand, when the Raman R value exceeds the above range, the crystallinity of the particle surface may decrease and its reactivity with the non-aqueous electrolyte solution may increase, leading to decrease in efficiency and increase in gas generation.

The measurement of the Raman spectrum is carried out by using a Raman spectrometer (for example, a Raman spectrometer manufactured by JASCO Corporation), in which a sample is dropped naturally to be charged into a measurement cell, and then, the surface of the sample in the cell is irradiated with argon ion laser light (or semiconductor laser light), while the cell is kept rotated in a plane perpendicular to the laser light. For the obtained Raman spectrum, the intensity I_{A} of a peak P_{A} near 1,580 cm⁻¹ and the intensity I_{B} of a peak P_{B} near 1,360 cm⁻¹ are measured to calculate an intensity ratio R (R = I_{B} / I_{A}). The Raman R value obtained from the measurement is defined as the Raman R value of the carbonaceous material of the present invention.

Conditions for the above-mentioned Raman measurement are as follows.
- laser wavelength: Ar ion laser 514.5 nm (semiconductor laser 532 nm)
- measurement range: from 1,100 cm⁻¹ to 1,730 cm⁻¹
- Raman R value: background removal
- smoothing procedure: simple average, five-point convolution

### (BET specific surface area)

The BET specific surface area of the carbonaceous material is a value of the specific surface area measured by the BET method, and it is usually 0.1 m²·g⁻¹ or more, preferably 0.7 m²·g⁻¹ or more, more preferably 1.0 m²·g⁻¹ or more, particularly preferably 1.5 m²·g⁻¹ or more, and is usually 100 m²·g⁻¹ or less, preferably 25 m²·g⁻¹ or less, more preferably 15 m²·g⁻¹ or less, particularly preferably 10 m²·g⁻¹ or less.

When the value of the BET specific surface area falls below this range, the material, used as a negative electrode material, tend to exhibit poor acceptability to lithium during charging, and therefore, to cause the precipitation of the lithium on the electrode surface, possibly resulting in decrease in the stability of the material. On the other hand, when the value exceeds this range, the material that is used as a negative electrode material tend to exhibit increased reactivity with the non-aqueous electrolyte solution, and therefore, to cause increased gas generation, possibly resulting in difficulty in providing a preferable battery.

The measurement of the specific surface area according to the BET method is carried out with a surface area meter (for example, full automatic surface area analyzer manufactured by Ohkura Riken Co,. Ltd.), for a sample preliminary dried under a nitrogen flow at 350°C for 15 minutes, according to the single point BET nitrogen adsorption method based on the gas flow method, using a nitrogen-helium mixed gas precisely adjusted so that the relative pressure value of nitrogen may become 0.3.

### <Structure and preparation method of negative electrode>

Any known method can be used for producing the electrode as long as the effects of the present invention are not significantly impaired. For example, the electrode can be formed from a negative electrode active material and a material added thereto, such as a binder, a solvent, and optionally, a thickener, a conductive material, and a filler, wherein these materials are combined into a slurry, which is then applied to and dried on a current collector, followed by press.

When an alloy material is used, a method is also used in which a thin film layer (negative electrode active material layer) containing the above-described negative electrode active material is formed by a method, such as vapor deposition, sputtering, and plating.

### (Electrode density)

The electrode structure of an electrode formed from the negative electrode active material does not have any particular limitation, and the density of the negative electrode active material present on the current collector is preferably 1 g·cm⁻³ or more, more preferably 1.2 g·cm⁻³ or more, particularly preferably 1.3 g·cm⁻³ or more, and preferably 2.2 g·cm⁻³ or less, more preferably 2.1 g·cm⁻³ or less, still more preferably 2.0 g·cm⁻³ or less, particularly preferably 1.9 g·cm⁻³ or less. When the density of the negative electrode active material present on the current collector exceeds the above range, particles of the negative electrode active material may be destroyed, leading to increase in the initial irreversible capacity or to the deterioration of charge and discharge characteristics in high current density owing to decrease in the permeability of the non-aqueous electrolyte solution into the vicinity of the interface between the current collector and the negative electrode active material. Further, when the density falls below the above range, the conductivity between the negative electrode active materials may decrease, causing increase in the battery resistance, and decrease in the capacity per unit.

### [3. Positive electrode]

### <Positive electrode active material>

A positive electrode active material (lithium transition metal-based compound) used for the positive electrode is described below.

### <Lithium transition metal-based compound>

The lithium transition metal-based compound is a compound having a structure capable of releasing and storing a Li ion, and examples thereof include sulfides, phosphate compounds, and lithium transition metal oxide composites. Examples of the sulfides include a compound having a two-dimensional layered structure such as TiS₂ and MoS₂, and a Chevrel compound having a strong three-dimensional skeleton structure represented by a general formula, MeₓMo₆S₈ (Me represents various transition metals including Pb, Ag, and Cu). Examples of the phosphate compounds include a compound belonging to the olivine structure, which is, in general, represented by LiMePO₄ (Me represents at least one kind of transition metal), and specifically include LiFePO₄, LiCoPO₄, LiNiPO₄, and LiMnPO₄. Examples of the lithium transition metal oxide composites include an oxide belonging to a spinel structure enabling three-dimensional diffusion and to a layered structure enabling the two-dimensional diffusion of lithium ions. Those having the spinel structure are generally represented by LiMe₂O₄ (Me represents at least one kind of transition metal), and specifically include LiMn₂O₄, LiCoMnO₄, LiNi_{0.5}Mn_{1.5}O₄, and LiCoVO₄. Those having the layered structure are generally represented by LiMeO₂ (Me represents at least one kind of transition metal). Specifically, they include LiCoO₂, LiNiO₂, LiNi₁₋ₓCoₓO₂, LiNi_{1-x-y}CoₓMn_{y}O₂, LiNi_{0.5}Mn_{0.5}O₂, Li_{1.2}Cr_{0.4}Mn_{0.4}O₂, Li_{1.2}Cr_{0.4}Ti_{0.4}O₂, and LiMnO₂.

### (Composition)

The lithium-containing transition metal compound is, for example, a lithium transition metal-based compound represented by the following composition formula (A) or (B) .
1) Case of lithium transition metal-based compound represented by following composition formula (A),

   Li₁₊ₓMO₂ (A)

   Note that x is usually 0 or more and 0.5 or less. M is an element consisting of Ni and Mn, or of Ni, Mn, and Co, and a molar ratio Mn / Ni is usually 0.1 or more and 5 or less. A molar ratio Ni / M is usually 0 or more and 0.5 or less. A molar ratio Co / M is usually 0 or more and 0.5 or less. Note that the rich portion of Li represented by x may be substituted with the transition metal site M.

In the above composition formula (A), the atomic ratio of the oxygen amount is described as 2 for convenience, but it may have nonstoichiometry to a certain extent. Furthermore, x in the above composition formula is a prepared composition of the lithium transition metal-based compound at its production stage. Normally, batteries on the market are aged after their assembling. Therefore, some amount of Li in the positive electrode may be lost as a result of charging and discharging. In this case, x after discharging to 3 V may be measured as -0.65 or more and 1 or less on the basis of compositional analysis.

The lithium transition metal-based compound is excellent in battery characteristics when sintered by high-temperature sintering carried out in an oxygen-containing gas atmosphere in order to enhance the crystallinity of the positive electrode active material.

The lithium transition metal-based compound represented by the composition formula (A) may also be a solid solution with Li₂MO₃, referred to as 213 layer, represented by the following general formula (A'),

*α* Li₂MO₃ · (1 - *α*) LiM'O₂. (A')

In the general formula, *α* is a number that satisfies 0 < *α* < 1.

The sign M represents at least one kind of metal element having an average oxidation number of 4⁺, and specifically, it represents at least one kind of metal element selected from the group consisting of Mn, Zr, Ti, Ru, Re, and Pt.

The sign M' represents at least one kind of metal element having an average oxidation number of 3⁺, preferably at least one kind of metal element selected from the group consisting of V, Mn, Fe, Co, and Ni, and more preferably at least one kind of metal element selected from the group consisting of Mn, Co, and Ni.

2) Case of lithium transition metal-based compound represented by the following general formula (B),

Li[LiₐM_{b}Mn_{2-b-a}]O₄₊*δ*. (B)

Here, M represents an element consisting of at least one kind of transition metal selected from Ni, Cr, Fe, Co, Cu, Zr, Al, and Mg.

The value of **b** is usually 0.4 or more and 0.6 or less.

The value of **b** within this range augments the energy density per unit weight in the lithium transition metal-based compound.

The value of **a** is usually 0 or more and 0.3 or less. Furthermore, **a** in the above composition formula is a prepared composition at the production stage of the lithium transition metal-based compound. In general, batteries on the market are aged after their assembling. Therefore, some amount of Li in the positive electrode may be lost as a result of charging and discharging. In this case, **a** after discharging to 3 V may be measured as -0.65 or more and 1 or less on the basis of compositional analysis.

The value of **a** within this range does not greatly impair the energy density per unit weight in the lithium transition metal-based compound and yields excellent load characteristic.

Further, the value of *δ* is usually in the range of ± 0.5.

The value of *δ* within this range augments the stability of the crystal structure of this lithium transition metal-based compound and yields the excellent cycle characteristic and storage at high temperature of a battery having an electrode manufactured using this compound.

Now, a detailed description will be made below for the chemical significance of the lithium composition in a lithium nickel manganese-based oxide composite that has the composition of the lithium transition metal-based compound.

In order to determine **a** and **b** in the composition formula of the above-described lithium transition metal-based compound, respective transition metals and lithium are analyzed with an Inductively Coupled Plasma Atomic Emission Spectrometer (ICP-AES), to obtain a ratio of Li / Ni / Mn.

From a structural point of view, the lithium corresponding to **a** is thought to replace the transition metal and occupy the same site. In this instance, the lithium corresponding to **a** makes the average valence value of M and manganese larger than 3.5 owing to the principle of charge neutrality.

The above-mentioned lithium transition metal-based compound may be substituted with fluorine and represented by LiMn₂O₄₋ₓF₂ₓ.

### (Blend)

Specific examples of the lithium transition metal-based compound having the above-described composition include Li₁₊ₓNi_{0.5}Mn_{0.5}O₂, Li₁₊ₓNi_{0.85}Co_{0.10}Al_{0.05}O₂, Li₁₊ₓNi_{0.33}Mn_{0.33}Co_{0.33}O₂, Li₁₊ₓNi_{0.45}Mn_{0.45}Co_{0.1}O₂, Li₁₊ₓMn_{1.8}Al_{0.2}O₄, and Li₁₊ₓMn_{1.5}Ni_{0.5}O₄. These lithium transition metal-based compounds may be used singly, or in a blend of two or more kinds thereof.

### (Introduction of heteroelement)

The lithium transition metal-based compound may contain a heteroelement introduced therein. The heteroelement is at least one selected from B, Na, Mg, Al, K, Ca, Ti, V, Cr, Fe, Cu, Zn, Sr, Y, Zr, Nb, Ru, Rh, Pd, Ag, In, Sb, Te, Ba, Ta, Mo, W, Re, Os, Ir, Pt, Au, Pb, La, Ce, Pr, Nd, Sm, Eu, Gd, Tb, Dy, Ho, Er, Tm, Yb, Lu, Bi, N, F, S, Cl, Br, I, As, Ge, P, Pb, Sb, Si, and Sn. These heteroelements may be incorporated in the crystal structure of the lithium transition metal-based compound, or may be unevenly distributed, as a single element or a compound, to the particle surface or to the grain boundary of the compound, rather than incorporated in the crystal structure.

### <Structure and preparation method of positive electrode for lithium secondary battery>

The positive electrode for lithium secondary battery is an electrode configured by a current collector and a positive electrode active material layer formed thereon that contains the powdered lithium transition metal-based compound for lithium secondary battery positive electrode material and a binder.

In general, the positive electrode active material layer is fabricated from a positive electrode material, a binder, and a material used as necessary, such as a conductive material and thickener, wherein these materials are mixed in dry process to form a sheet, which is then pressure-bonded to a positive electrode current collector, or these materials are dissolved or dispersed in a liquid medium to form a slurry, which is then applied to and dried on the positive electrode current collector.

Examples of a material usually used as the material of the positive electrode current collector include a metal material, such as aluminum, stainless steel, nickel plating, titanium, tantalum, and a carbon material such as carbon cloth and carbon paper. Further, when the metal material is used, examples of the shape of the positive electrode current collector include a metal foil, a metal cylinder, a metal coil, a metal plate, a metal thin film, an expanded metal, a punched metal, a metal foam, and the like, and when the carbon material is used, they include a carbon plate, a carbon thin film, a carbon cylinder, and the like. The thin film may be formed into a mesh-like shape as appropriate.

When the thin film is used as the positive electrode current collector, its thickness is arbitrary, and is usually preferably in the range of 1 µm or more and 100 mm or less. When the film is thinner than the above range, it may not exhibit sufficient strength required for current collectors, whereas when it is thicker than the above range, it may exhibit worsened handling ability.

The binder used for producing the positive electrode active material layer does not have any particular limitation, and when used in coating methods, any material may be used as long as it is stable in a liquid medium used for manufacturing the electrode, and specific examples thereof include a resin-based polymer, such as polyethylene, polypropylene, polyethylene terephthalate, polymethyl methacrylate, aromatic polyamide, cellulose, and nitrocellulose, a rubber polymer, such as SBR (styrene/butadiene rubber), NBR (acrylonitrile/butadiene rubber), fluororubber, isoprene rubber, butadiene rubber, and ethylene/propylene rubber, a thermoplastic elastomeric polymer, such as styrene/butadiene/styrene block copolymer and a hydrogenated product thereof, EPDM (ethylene/propylene/diene terpolymer), styrene/ethylene/butadiene/ethylene copolymer, and styrene/isoprene styrene block copolymer and hydrogenated products thereof, a soft resin-like polymer, such as syndiotactic 1,2-polybutadiene, polyvinyl acetate, ethylene-vinyl acetate copolymer, and propylene/α-olefin copolymer, a fluorinated polymer, such as polyvinylidene fluoride, and polytetrafluoroethylene, and polytetrafluoroethylene/ethylene copolymer, and a polymeric composition having ionic conductivity from an alkali metal ion (particularly lithium ion). These materials may be used singly or in any combination of two or more kinds thereof in any combination ratio.

The proportion of the binder in the positive electrode active material layer is usually 0.1% by mass or more and 80% by mass or less. When the proportion of the binder is too low, the binder may not be able to support the positive electrode active material sufficiently, leading to the insufficient mechanical strength of the positive electrode, thus resulting in deterioration of battery performances such as the cycle characteristic, but on the other hand, when the proportion is too high, the battery capacity and the conductivity may be worsen.

In general, the positive electrode active material layer contains a conductive material in order to enhance its conductivity. Specific examples of the conductive material include a metal material such as copper and nickel, and a carbon material, such as graphite such as natural graphite and artificial graphite, carbon black such as acetylene black, and amorphous carbon such as needle coke. These materials may be used singly or in any combination of two or more kinds thereof in any combination ratio. The proportion of the conductive material in the positive electrode active material layer is usually 0.01% by mass or more and 50% by mass or less. When the proportion of the conductive material is too low, the conductivity may be insufficient, and conversely, when it is too high, the battery capacity may decrease.

The liquid medium for forming a slurry does not have any particular limitation on its type as long as it is a solvent capable of dissolving or dispersing the lithium transition metal-based compound powder that is a positive electrode material, the binder, and the material used necessary such as conductive material and thickener, and either aqueous solvents or organic solvents may be used. Examples of the aqueous solvents include water and alcohol, and examples of the organic solvents include N-methylpyrrolidone (NMP), dimethylformamide, dimethylacetamide, methyl ethyl ketone, cyclohexanone, methyl acetate, methyl acrylate, diethyl triamine, *N,N-*dimethylaminopropylamine, ethylene oxide, tetrahydrofuran (THF), toluene, acetone, dimethyl ether, dimethylacetamide, hexamethylphosphoramide, dimethylsulfoxide, benzene, xylene, quinoline, pyridine, methylnaphthalene, and hexane. In particular, when an aqueous solvent is used, a dispersant along with the thickener is added to the solvent, which is then slurried by using a latex such as SBR. These solvents may be used singly, or in any combination of two or more kinds thereof in any combination ratio.

The proportion of the content of the lithium transition metal-based compound powder as the positive electrode material in the positive electrode active material layer is usually 10% by mass or more and 99.9% by mass or less. When the proportion of the lithium transition metal-based compound powder in the positive electrode active material layer is too large, the strength of the positive electrode tends to be insufficient, and when it is too small, the capacity may be insufficient.

The thickness of the positive electrode active material layer is usually about 10 to 200 µm.

The electrode density of the positive electrode after press is usually 2.2 g/cm³ or more and 4.2 g/cm³ or less.

The positive electrode active material layer obtained through coating and drying is preferably densified by roller press or the like in order to increase the packing density of the positive electrode active material.

### [4. Separator]

A separator is usually interposed between the positive electrode and the negative electrode in order to prevent short circuit. In this case, the non-aqueous electrolyte solution of the present invention is usually used in a state impregnated into this separator.

The material and shape of the separator does not have any particular limitation and any known separator can be adopted as long as the effects of the present invention are not significantly impaired. In particular, resins, glass fibers,' and inorganic materials, which is formed from a material stable in the non-aqueous electrolyte solution of the present invention are used, and those in a form of porous sheet or a nonwoven fabric excellent in liquid retentivity are preferably used.

Examples which may be used as the material of the resin or glass fiber separator include, for example, a polyolefin such as polyethylene and polypropylene, aromatic polyamide, polytetrafluoroethylene, polyethersulfone, and glass filter. In particular, the glass filter and the polyolefin are preferable, and the polyolefin is more preferable. These materials may be used singly, or in any combination of two or more kinds thereof in any combination ratio.

The thickness of the separator is arbitrary, and it is usually 1 µm or more, preferably 5 µm or more, more preferably 10 µm or more, and usually 50 µm or less, preferably 40 µm or less, more preferably 30 µm or less. When the separator is too thinner than the above range, the insulation and mechanical strength may decrease. When it is too thick, battery performances such as rate characteristic may be deteriorated, and in addition, the energy density of the non-aqueous electrolyte solution secondary battery as a whole may decrease.

Further, when a material such as the porous sheet or the nonwoven fabric is used as the separator, the porosity of the separator is arbitrary, and it is usually 20% or more, preferably 35% or more, more preferably 45% or more, and usually 90% or less, preferably 85% or less, more preferably 75% or less. When the porosity is too small, the film resistance tends to increase, leading to the deterioration of the rate characteristic. On the other hand, when it exceeds the above range, the mechanical strength of the separator tends to decrease, leading to decrease in the insulation characteristic.

The average pore size of the separator is also arbitrary, and it is usually 0.5 µm or less, preferably 0.2 µm or less, and is usually 0.05 µm or more. When the average pore size exceeds the above range, short circuit tends to occur. In addition, when it falls below the above range, the film resistance may increase, leading to the deterioration of the rate characteristic.

On the other hand, examples as the inorganic material include, for example, an oxide such as alumina and silicon dioxide, a nitride such as aluminum nitride and silicon nitride, a sulfate such as barium sulfate and calcium sulfate, and those which are in a particulate or fabric form are used.

Examples in a thin film-like form, such as a nonwoven fabric, a woven fabric, and a microporous film are used. In the thin film-like form, materials having a pore diameter of 0.01 to 1 µm and a thickness of 5 to 50 µm are preferably used. In addition to a separator in the independent thin film-like form as described above, a separator can be used which is configured by a positive electrode and/or a negative electrode and a composite porous layer formed on the electrode surface by using a resin binder, wherein the layer contains particles of the above inorganic material. Examples of the layer include a porous layer of alumina particles formed on the both sides of a positive electrode by using a fluororesin as a binder, wherein the alumina particles have a 90% particle size of less than 1 µm.

Characteristics of the separator in the non-aqueous electrolyte solution secondary battery can be grasped by a Gurley value. The Gurley value indicates an extent of difficulty for air to pass through a film in its thickness direction and is represented by the number of seconds required for 100 ml of air to pass through the film, and therefore a smaller value means larger easiness in the passing, and a larger value means larger difficulty in the passing. In other words, the smaller value means the better communicability of the film in its thickness direction, and the larger value means the poor communicability in its thickness direction. The communicability is the extent of the connection between pores in the film thickness direction. When the Gurley value of the separator of the present invention is low, the separator can be used for various purposes. For example, when the separator is used as a separator for non-aqueous lithium secondary batteries, a low Gurley value is preferable because it means the better mobility of lithium ions and excellence in the battery performance. The Gurley value of the separator is arbitrary, and it is preferably from 10 to 1,000 sec/100 ml, more preferably from 15 to 800 sec/100 ml, still more preferably from 20 to 500 sec/100 ml. When the Gurley value is 1,000 sec/100 ml or less, electric resistance is substantially low, which is preferable for separators.

### [5. Battery design]

### <Electrode group>

The electrode group may have either of a laminate structure configured by a positive electrode plate, a negative electrode plate, and the above described separator interposed between the electrodes, or a spirally wound structure configured by a positive electrode plate, a negative electrode plate, and the separator interposed between the electrodes. The proportion of the volume of the electrode group to the internal volume of a battery (hereinafter referred to as electrode group occupancy) is usually 40% or more, preferably 50% or more, and is usually 90% or less, preferably 80% or less.

When the electrode group occupancy falls below the above range, the battery capacity decreases. Further, when the occupancy exceeds the above range, the vacant space becomes small, leading to temperature raise of the battery, which in turn leads to increase in the internal pressure caused by the expansion of the battery members or by increase in the vapor pressure of the liquid component of the electrolyte, possibly resulting in the deterioration of various performances including the charge/discharge repetition performance and the high-temperature storage, and moreover resulting in the action of a gas discharge valve for releasing the internal pressure to the outside.

### <Outer casing>

The material of the outer casing does not have any particular limitation as long as it is a material stable against a non-aqueous electrolyte solution to be used. Specifically, a metal such as a nickel-plated steel plate, stainless steel, aluminum or an aluminum alloy, and a magnesium alloy, or a film of a lamination of resin and aluminum foil (laminated film) is used. From the viewpoint of weight reduction, a metal such as aluminum or an aluminum alloy, or the laminated film is preferably used.

Examples of a structure for the outer casing based on metals include a closed structure sealed by welding the metals by laser welding, resistance welding, and ultrasonic welding, and a caulked structure using the above metals with resin gaskets interposed therebetween. Examples of a structure for the outer case based on the above laminated film include a closed structure sealed by resin layers thermally fused to each other. In order to improve sealing characteristics, a resin different from the resin used for the laminated film may be interposed between the resin layers. In particular, when the resin layers are thermally sealed, with the collector terminal interposed therebetween, to form a sealed structure, a juncture of metal and resin occurs, and therefore, an intervening resin is preferably used which is a resin having a polar group or a modified resin having an introduced polar group.

### <Protective element>

A protective element can be used, the examples of which include PTC (Positive Temperature Coefficient) that increases resistance when abnormal heat generation or excessive current flow occurs, a thermal fuse, a thermistor, a valve (current cutoff valve) that shuts off a current flow through a circuit caused by rapid increase in the internal pressure or internal temperature of a battery during abnormal heat generation. The above-described protective element is preferably an element selected from protective elements for high current that do not operate under normal use, and more preferable is that the battery is designed not to cause abnormal heat generation or thermal runaway without any protective element.

### <Outer body>

The non-aqueous electrolyte solution secondary battery of the present invention is usually constituted by the non-aqueous electrolyte solution, the negative electrode, the positive electrode, the separator, and the like, and an outer body housing these components. The outer body does not have any particular limitation, and any known outer body can be adopted as long as the effects of the present invention are not significantly impaired. The material of the outer body is arbitrary, but specifically, a material such as nickel-plated iron, stainless steel, aluminum or an alloy thereof, nickel, and titanium is used in general.

In addition, the shape of the outer body is also arbitrary, and may be any shape, such as a cylindrical shape, a square shape, a laminated shape, a coin shape, and a large sized shape.

### <Battery voltage>

The non-aqueous electrolyte solution secondary battery of the present invention is usually used at a battery voltage of 4.3 V or higher. The battery voltage is preferably 4.3 V or higher, more preferably 4.35 V or higher, and most preferably 4.4 V or higher. This is because when the battery voltage is increased, the energy density of the battery can be augmented. On the other hand, when the battery voltage is elevated, a problem occurs that the potential of the positive electrode is elevated to enhance a side reaction on the surface of the positive electrode. The above problem can be solved by using the electrolyte solution and the battery of the present invention, but when the voltage is too high, the side reaction on the surface of the positive electrode becomes too excessive, thus causing deterioration of battery characteristics. Therefore, the upper limit of the battery voltage is preferably 5 V or less, more preferably 4.8 V or less, most preferably 4.6 V or less.

### EXAMPLES

Hereinafter, the present invention will be described in more detail with reference to Examples and Comparative Examples, but the present invention is not limited to these Examples unless it departs from the gist of the invention.

### <Preparation of non-aqueous electrolyte solution>

### <Example 1-1>

In a dry argon atmosphere, ethylene carbonate (hereinafter referred to as EC), ethyl methyl carbonate (hereinafter EMC) and diethyl carbonate (hereinafter DEC) were mixed so as to be 30% by volume, 40% by volume, and 30% by volume, respectively, to form a non-aqueous solvent, into which was dissolved LiPF₆ so as to be 1.2 M, and were added 2% by mass of vinylene carbonate and 2% by mass of fluoroethylene carbonate. Further, 0.3% by mass of a compound 2-10 was added to prepare a non-aqueous electrolyte solution.

### <Comparative Example 1-1>

A non-aqueous electrolyte solution was prepared in the same manner as in Example 1-1, except that a non-aqueous electrolyte solution without the compound 2-10 was used in the non-aqueous electrolyte solution of Example 1-1.

### <Comparative Example 1-2>

A non-aqueous electrolyte solution was prepared in the same manner as in Example 1-1, except that 0.3% by mass of a compound A (not included in the present invention) represented by the following formula was added instead of the compound 2-10 in the non-aqueous electrolyte solution of Example 1-1.

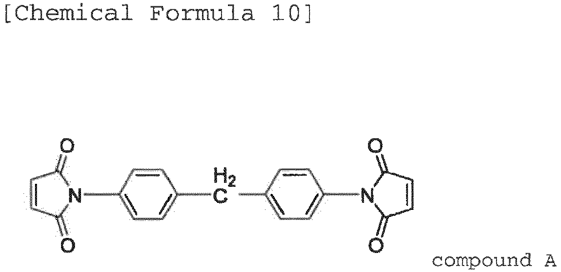

### <Preparation of negative electrode>

To 98 parts by mass of graphite powder as a negative electrode active material were added as a thickener, and binders, one part by mass of an aqueous dispersion of sodium carboxymethyl cellulose and one part by mass of an aqueous dispersion of styrene-butadiene rubber, respectively, and mixed by using a disperser, to form a slurry. The resultant slurry was applied to one side of copper foils, dried, and pressed to prepare negative electrodes. The prepared negative electrodes were used after being dried under reduced pressure at 60°C for 12 hours.

### <Production of positive electrode>

To 96.8 parts by mass of lithium cobalt oxide as a positive electrode active material were added 1.6 parts by mass of a conductive aid and 1.6 parts by mass of a binder (pVDF), and mixed by using a disperser, to form a slurry. The resultant slurry was applied to both sides of aluminum foils, dried, and pressed to prepare positive electrodes. The prepared positive electrodes were used after being dried under reduced pressure at 80°C for 12 hours.

### <Preparation of battery>

Each of battery elements was fabricated by laminating the positive electrode, the negative electrodes, and separators made of polyethylene, in the order of the negative electrode, the separator, the positive electrode, the separator, and the negative electrode. Each of the battery elements was inserted into a bag made of a laminate film of aluminum (thickness: 40 µm) and a resin layer coating the both surface of the aluminum, such that the terminals of the positive and negative electrodes allowed to protrude out, and then, 0.4 mL of the non-aqueous electrolyte solutions of Examples and Comparative Examples were each injected to the bags, which were then vacuum-sealed to prepare sheet-like batteries. Further, in order to enhance adhesion between the electrodes, the sheet-like batteries were sandwiched between glass plates and pressurized.

### <Test of characteristic evaluation>

### Test 1. Test of high temperature storage at 60°C

Each of the batteries prepared as described above was conditioned by being charged to 4.4 V and discharged to 3 V at 25°C until its capacity was stabilized. Then, the battery charged to 4.4 V was left for 10 days in an environment of 60°C. A residual capacity ratio (%) in this instance was measured. Note that a capacity after discharge to 3 V at 0.2 C at 25°C after the test is defined as a residual capacity, and the ratio of the residual capacity to the capacity before the test is defined as the residual capacity ratio.

### [Table 1]

**Table 1**

| | Bismaleimide compound of the present invention | other compounds | residual capacity ratio |
|---|---|---|---|
| | (mass%) | (mass%) | (%) |
| Example 1-1 | compound 2-10 (0.3) | - | 79.7 |
| Comparative Example 1-1 | - | - | 76.4 |
| Comparative Example 1-2 | - | compound A (0.3) | 77.7 |

As is apparent from the above Table 1, the use of the electrolyte solution of the present invention significantly improved the residual capacity ratio as in Example 1-1. On the other hand, although the used of the electrolyte solution containing the compound A that was not the electrolyte solution of the present invention improved the residual capacity ratio, the obtained improvement effect was not comparable to that in the use of the electrolyte solution of the present invention.

### <Example 2-1>

In a dry argon atmosphere, ethylene carbonate (hereinafter referred to as EC), ethyl methyl carbonate (hereinafter, EMC), and diethyl carbonate (hereinafter, DEC) were mixed, so as to be 30% by volume, 40% by volume, and 30% by volume, respectively, to form a non-aqueous solvent, into which was dissolved LiPF₆ so as to be 1.2 M, and were added 2 mass% of vinylene carbonate, 2 mass% of fluoroethylene carbonate, and 1 mass% of adiponitrile. Further, 0.3% by mass of the compound 2-10 was added to prepare a non-aqueous electrolyte solution. A sheet-like battery was fabricated in the same manner as in Example 1-1 except that this non-aqueous electrolyte solution was used.

### <Comparative Example 2-1>

A non-aqueous electrolyte solution was prepared in the same manner as in Example 2-1, except that a non-aqueous electrolyte solution without the compound 2-10 was used in the non-aqueous electrolyte solution of Example 2-1. A sheet-like battery was fabricated in the same manner as in Example 2-1 except that this non-aqueous electrolyte solution was used.

### Test 2. Cycle test

The battery prepared as described above was conditioned by being charged to 4.4 V and discharged to 3 V at 25°C until its capacity was stabilized. Then, a cycle test was performed in which 500 cycles of charging to 4.4 V and discharging to 3 V were repeated in an environment of 45°C at a current value of 0.7 C (1 C indicates a current value with which one hour is required for charging or discharging). The ratio of the discharge capacity at the 200th cycle to the discharge capacity at the first cycle was defined as 200-cycle capacity retention ratio (%). The ratio of the discharge capacity at the 500th cycle to the discharge capacity at the first cycle was defined as 500-cycle capacity retention ratio (%).

### [Table 2]

**Table 2**

| | bismaleimide compound of the present invention (mass%) | 200-cycle capacity retention ratio (%) | 500-cycle capacity retention ratio (%) |
|---|---|---|---|
| Example 2-1 | compound 2-10 (0.3) | 83.3 | 68.8 |
| Comparative Example 2-1 | - | 82.4 | 66.6 |

As is apparent from the above Table 2, the use of the electrolyte solution of the present invention yielded an effect of improving the cycle capacity retention ratio.

### <Example 3-1>

In a dry argon atmosphere, ethylene carbonate (hereinafter referred to as EC), ethyl methyl carbonate (hereinafter EMC), and diethyl carbonate (hereinafter DEC) were mixed, so as to be 30% by volume, 40% by volume, and 30% by volume, respectively, to form a non-aqueous solvent, into which was dissolved LiPF₆ so as to be 1.2 M, and were added 2% by mass of vinylene carbonate, 2% by mass of fluoroethylene carbonate, and 1% by mass of adiponitrile. Further, 0.3% by mass of the compound 2-10 was added to prepare a non-aqueous electrolyte solution. A sheet-like battery was fabricated in the same manner as in Example 1-1 except that this non-aqueous electrolyte solution was used.

### <Example 3-2>

A non-aqueous electrolyte solution was prepared in the same manner as in Example 3-1, except that the content of the compound 2-10 was changed to 0.5% by mass in the non-aqueous electrolyte solution of Example 3-1. A sheet-like battery was fabricated in the same manner as in Example 3-1, except that this non-aqueous electrolyte solution was used.

### <Comparative Example 3-1>

A non-aqueous electrolyte solution was prepared in the same manner as in Example 3-1, except that a non-aqueous electrolyte solution without the compound 2-10 was used in the non-aqueous electrolyte solution of Example 3-1. A sheet-like battery was fabricated in the same manner as in Example 3-1 except that this non-aqueous electrolyte solution was used.

### <Comparative Example 3-2>

A non-aqueous electrolyte solution of Example 3-1 was prepared in the same manner as in Example 3-1, except that in the electrolyte solution of Example 3-1, 0.3% by mass of the compound A used in Comparative Example 1-2 was added instead of the compound 2-10. A sheet-like battery was fabricated in the same manner as in Example 3-1 except that this non-aqueous electrolyte solution was used.

### Test 3. Test of high temperature storage at 80°C

The battery prepared as described above was conditioned by being charged to 4.4 V and discharged to 3 V at 25°C until its capacity was stabilized. Then, the battery charged to 4.4 V was left for 3 days in an environment of 80°C. A gas generation ratio (%) and a residual capacity ratio (%) in this instance were measured. The gas generation ratio is defined as a ratio (%) of the amount of gas generated in each test to the amount, which is set to be 100, of gas generated in Comparative Example 3-1, wherein the amounts are obtained by using the Archimedes method (a smaller value of the ratio is preferable). Further, the capacity after discharging to 3 V at 0.2 C at 25°C after the test is defined as a residual capacity, and the ratio of the residual capacity to the capacity before the test is defined as the residual capacity ratio.

### [Table 3]

**Table 3**

| | bismalcimidc compound of the present invention (mass%) | other compounds (mass%) | Gas generation ratio (%) | residual capacity ratio (%) |
|---|---|---|---|---|
| Example 3-1 | compound 2-10 (0.3) | - | 83.1 | 71.3 |
| Example 3-2 | compound 2-10 (0.5) | - | 86.9 | 71.7 |
| Comparative Example 3-1 | - | - | 100.0 | 70.4 |
| Comparative Example 3-2 | - | compound A (0.3) | 88.2 | 71.1 |

As is apparent from the above Table 3, the use of the electrolyte solutions of the present invention yielded effects of reducing the gas generation ratio, and effects of improving the residual capacity ratio were also confirmed. On the other hand, although Comparative Example 3-2 using an electrolyte solution that was not the present invention exhibited a similar improvement effect, it was not comparable to that of the electrolyte solution of the present invention.

### <Example 4-1>

In a dry argon atmosphere, ethylene carbonate (hereinafter referred to as EC), ethyl methyl carbonate (hereinafter EMC), and diethyl carbonate (hereinafter DEC) were mixed, so as to be 30% by volume, 40% by volume, and 30% by volume, respectively, to form a non-aqueous solvent, into which was dissolved LiPF₆ so as to be 1.2 M, and were added 2% by mass of vinylene carbonate and 2% by mass of fluoroethylene carbonate. Further, 0.3% by mass of a compound 2-10 was added to prepare a non-aqueous electrolyte solution. A sheet-like battery was fabricated in the same manner as in Example 1-1 except that this non-aqueous electrolyte solution was used.

### <Comparative Example 4-1>

A non-aqueous electrolyte solution was prepared in the same manner as in Example 4-1, except that a non-aqueous electrolyte solution without the compound 2-10 was used in the non-aqueous electrolyte solution of Example 4-1. A sheet-like battery was fabricated in the same manner as in Example 4-1 except that this non-aqueous electrolyte solution was used.

### <Comparative Example 4-2>

A non-aqueous electrolyte solution was prepared in the same manner as in Example 4-1, except that a compound B (not included in the present invention) was used instead of the compound 2-10 in the non-aqueous electrolyte solution of Example 4-1. A sheet-like battery was fabricated in the same manner as in Example 4-1, except that this non-aqueous electrolyte solution was used.

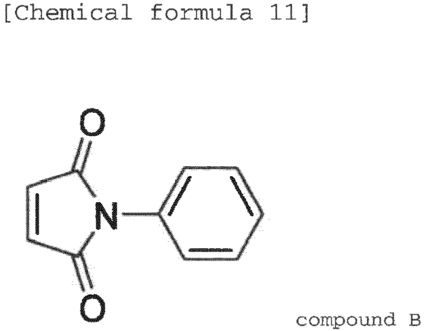

### <Comparative Example 4-3>

A non-aqueous electrolyte solution was prepared in the same manner as in Example 4-1, except that a compound C (not included in the present invention) was used instead of the compound 2-10 in the non-aqueous electrolyte solution of Example 4-1. A sheet-like battery was fabricated in the same manner as in Example 4-1 except that this non-aqueous electrolyte solution was used.

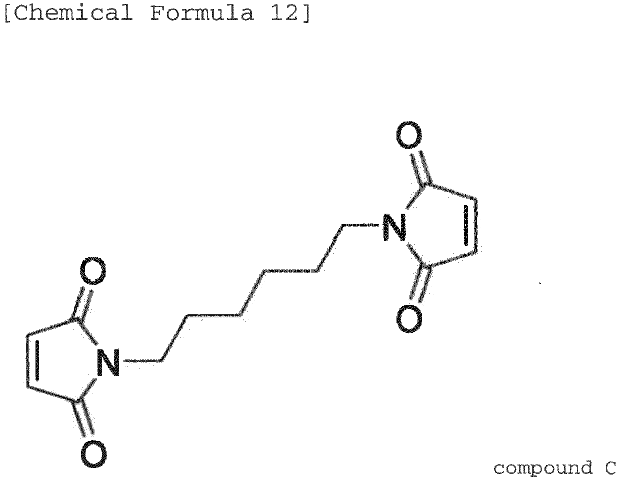

### <Comparative Example 4-4>

A non-aqueous electrolyte solution was prepared in the same manner as in Example 4-1, except that in the non-aqueous electrolyte solution of Example 4-1, a compound D (not included in the present invention) was used instead of the compound 2-10. A sheet-like battery was fabricated in the same manner as in Example 4-1, except that this non-aqueous electrolyte solution was used.

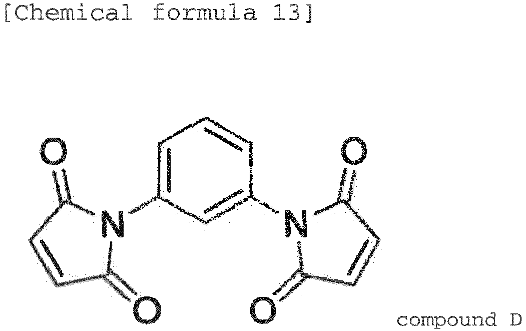

### <Comparative Example 4-5>

A non-aqueous electrolyte solution was prepared in the same manner as in Example 4-1, except that in the non-aqueous electrolyte solution of Example 4-1, the compound A (not included in the present invention) was used instead of the compound 2-10. A sheet-like battery was fabricated in the same manner as in Example 4-1, except that this non-aqueous electrolyte solution was used.

### Test 4. Test of high temperature storage at 85°C

The battery prepared as described above was conditioned by being charged to 4.4 V and discharged to 3 V at 25°C until its capacity was stabilized. Then, the battery charged to 4.4 V was left for 6 hours in an environment of 85°C. A gas generation ratio (%) in this instance was measured. The gas generation ratio is defined as a ratio (%) of the amount of gas generated in each test to the amount, which is set to be 100, of gas generated in Comparative Example 4-1, wherein the amounts are determined using the Archimedes method (a smaller value of the ratio is preferable).

### Test 5. Test of load discharge

The battery prepared as described above was conditioned by being charged to 4.4 V and discharged to 3 V at 25°C until its capacity was stabilized. Then, the battery charged to 4.4 V was discharged to 3 V at a current of 0.2 C in an environment of 25°C (the capacity in this instance is defined as 0.2 C capacity). Again, the battery charged to 4.4 V was discharged to 3 V at a current of 0.5 C in an environment of 25°C (the capacity in this instance is defined as 0.5 C capacity). For this case, a load characteristic was obtained which was defined as a value of 0.5 C capacity / 0.2 C capacity × 100 (%).

### [Table 4]

**Table 4**

| | bismaleimide compound of the present invention | other compounds | gas generation ratio | load characteristic |
|---|---|---|---|---|
| | (mass%) | (mass%) | (%) | (%) |
| Example 4-1 | compound 2-10 (0.3) | - | 84.8 | 97.66 |
| Comparative Example 4-1 | - | - | 100.0 | 97.59 |
| Comparative Example 4-2 | - | compound B (0.3) | 112.1 | 97.59 |
| Comparative Example 4-3 | - | compound C (0.3) | 85.9 | 97.55 |
| Comparative Example 4-4 | - | compound D (0.3) | 130.3 | 97.74 |
| Comparative Example 4-5 | - | compound A (0.3) | 122.2 | 97.71 |

As is apparent from Table 4, in Example 4-1 using the electrolyte solution of the present invention, not only the gas generation ratio was able to be suppressed significantly, but also the load characteristic was able to be improved as compared to Comparative Example 4-1. On the other hand, in Comparative Example 4-2, Comparative Example 4-4, and Comparative Example 4-5, using maleimide and not falling within the electrolytic solution of the present invention, the load characteristic was either the same as or superior to that in Comparative Example 4-1, but the gas generation ratio increased significantly. Similarly, in Comparative Example 4-3 using maleimide that was not the electrolyte solution of the present invention, the gas generation ratio decreased as compared to that in Comparative Example 4-1, but the effect was smaller than that of the electrolyte solution of the present invention, and moreover, the load characteristic became worse. From these results, it can be said that the specific compound of the present invention is necessary in order to simultaneously achieve the suppression of the gas generation and the improvement of the load characteristic.

### [Industrial Applicability]

According to the non-aqueous electrolyte solution of the present invention, a non-aqueous electrolyte solution secondary battery with high energy density can be manufactured which achieves the suppressed decomposition of the electrolyte solution of the non-aqueous electrolyte solution secondary battery, the suppressed gas generation when used under high temperature environment, the improved residual capacity of the battery, and the improved cycle characteristic thereof, and further, is excellent in load discharge characteristic (dischargeable at high rate). Accordingly, it can be suitably used in various fields such as electronic devices using non-aqueous electrolyte solution secondary batteries.

Applications of the non-aqueous electrolyte solution secondary battery of the present invention do not have any particular limitation, and the battery can be used for various known applications. Specific examples thereof include notebook computers, pen-input personal computers, mobile personal computers, electronic book players, mobile phones, portable faxes, portable copy machines, portable printers, headphone stereo cassette players, video movies, LCD televisions, handy cleaners, portable CD players, mini disk players, transceivers, electronic organizers, calculators, memory cards, portable tape recorders, radios, backup power supplies, motors, cars, motorcycles, motorized bicycles, bicycles, lighting equipment, toys, game machines, clocks, electric tools, strobes, cameras, large household storage batteries, and lithium ion capacitors.

## Claims

1. A non-aqueous electrolyte solution for use in a non-aqueous electrolyte solution secondary battery comprising a positive electrode comprising a positive electrode active material capable of absorbing and releasing a metal ion and a negative electrode comprising a negative electrode active material capable of absorbing and releasing a metal ion, which solution comprises a compound represented by the following formula (1), 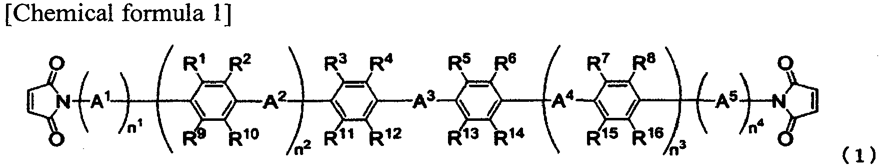
wherein, in the formula (1), each of R¹ to R¹⁶ independently represents any one of a hydrogen atom, a halogen atom, a hydrocarbon group, a group represented by -O-L¹, and a group represented by -SO₂-L²,
L¹ and L² represent a hydrocarbon group,
each of A¹ to A⁵ independently represents a divalent hydrocarbon group, a hetero atom, or a group having a hetero atom, and
each of n¹ to n⁴ represents an integer of 0 or more, with the proviso that when all of n¹ to n⁴ are 0, at least one of R³ to R⁶ and R¹¹ to R¹⁴ represents a group other than a hydrogen atom, and
wherein the compound represented by the formula (1) is a compound represented by the following formula (2), 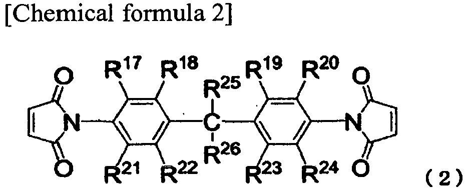
wherein, in the formula (2), each of R¹⁷ to R²⁶ independently represents any one of a hydrogen atom, a halogen atom, a hydrocarbon group, a group represented by -O-L¹, and a group represented by -SO₂-L²,
L¹ and L² each represent a hydrocarbon group, and
at least one of R¹⁷ to R²⁴ represents a group other than a hydrogen atom.

2. The non-aqueous electrolyte solution according to claim 1, wherein a content of the compound represented by the formula (1) is 0.01% by mass or more and 5% by mass or less in the non-aqueous electrolyte solution.

3. The non-aqueous electrolyte solution according to claim 1 or 2, wherein in the compound represented by the formula (1), R¹ to R¹⁶ are a hydrogen atom or an alkyl group.

4. The non-aqueous electrolyte solution according to any one of claims 1 to 3, comprising a water content of 40 ppm by mass or less.

5. The non-aqueous electrolyte solution according to any one of claims 1 to 4, further comprising at least one of a cyclic carbonate comprising a carbon-carbon unsaturated bond and a cyclic carbonate comprising a fluorine atom.

6. The non-aqueous electrolyte solution according to any one of claims 1 to 5, further comprising a nitrile compound.

7. A non-aqueous electrolyte solution secondary battery comprising a positive electrode comprising a positive electrode active material capable of absorbing and releasing a metal ion, a negative electrode comprising a negative electrode active material capable of absorbing and releasing a metal ion, and a non-aqueous electrolyte solution, which battery uses the non-aqueous electrolyte solution according to any one of claims 1 to 6.

## Patentansprüche

1. Eine nichtwässrige Elektrolytlösung zur Verwendung in einer Sekundärbatterie mit nichtwässriger Elektrolytlösung, umfassend eine positive Elektrode, die ein Positivelektroden-Aktivmaterial umfasst, das ein Metallion absorbieren und freisetzen kann und eine negative Elektrode, die ein Negativelektroden-Aktivmaterial umfasst, das ein Metallion absorbieren und freisetzen kann, wobei die Lösung eine Verbindung dargestellt durch die nachstehende Formel (1) umfasst, 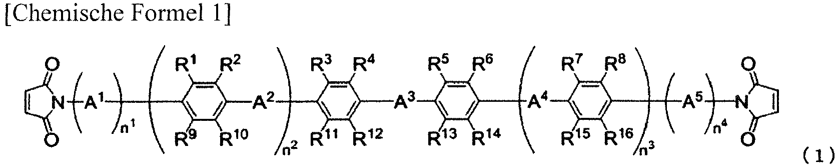
wobei in der Formel (1) jedes aus R¹ bis R¹⁶ unabhängig voneinander eines aus einem Wasserstoffatom, einem Halogenatom, einer Kohlenwasserstoffgruppe, einer Gruppe dargestellt durch -O-L¹ und einer Gruppe dargestellt durch -SO₂L², darstellt,
L¹ und L² eine Kohlenwasserstoffgruppe darstellen,
jedes aus A¹ bis A⁵ unabhängig eine zweiwertige Kohlenwasserstoffgruppe, ein Heteroatom oder eine Gruppe, die ein Heteroatom aufweist, darstellt, und
jedes aus n¹ bis n⁴ eine ganze Zahl von 0 oder mehr darstellt, mit der Maßgabe, dass, wenn alle aus n¹ bis n⁴ gleich 0 sind, mindestens eines aus R³ bis R⁶ und R¹¹ bis R¹⁴ eine von einem Wasserstoffatom verschiedene Gruppe darstellt, und
wobei die Verbindung, die durch die Formel (1) dargestellt ist, eine durch die nachstehende Formel (2) dargestellte Verbindung ist, 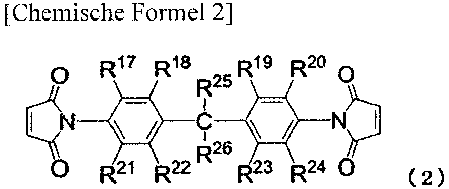
wobei in der Formel (2) jedes aus R¹⁷ bis R²⁶ unabhängig eines aus einem Wasserstoffatom, einem Halogenatom, einer Kohlenwasserstoffgruppe, einer Gruppe, dargestellt durch -O-L¹ und einer Gruppe, dargestellt durch -SO₂-L², darstellt,
L¹ und L² jeweils eine Kohlenwasserstoffgruppe darstellen, und
mindestens eines aus R¹⁷ bis R²⁴ eine von einem Wasserstoffatom verschiedene Gruppe darstellt.

2. Die nichtwässrige Elektrolytlösung nach Anspruch 1, wobei ein Gehalt der durch die Formel (1) dargestellten Verbindung 0,01 Massen-% oder mehr und 5 Massen-% oder weniger in der nichtwässrigen Elektrolytlösung beträgt.

3. Die nichtwässrige Elektrolytlösung nach Anspruch 1 oder 2, wobei in der durch Formel (1) dargestellten Verbindung R¹ bis R¹⁶ ein Wasserstoffatom oder eine Alkylgruppe sind.

4. Die nichtwässrige Elektrolytlösung nach einem der Ansprüche 1 bis 3, umfassend einen Wassergehalt von 40 Massen-ppm oder weniger.

5. Die nichtwässrige Elektrolytlösung nach einem der Ansprüche 1 bis 4, weiter umfassend mindestens eines aus einem cyclischen Carbonat, das eine ungesättigte Kohlenstoff-Kohlenstoff-Bindung umfasst und ein cyclisches Carbonat, das ein Fluoratom umfasst.

6. Die nichtwässrige Elektrolytlösung nach einem der Ansprüche 1 bis 5, weiter umfassend eine Nitrilverbindung.

7. Eine Sekundärbatterie mit nichtwässriger Elektrolytlösung, umfassend eine positive Elektrode, die ein Positivelektroden-Aktivmaterial umfasst, das ein Metallion absorbieren und freisetzen kann, eine negative Elektrode, die ein Negativelektroden-Aktivmaterial umfasst, das ein Metallion absorbieren und freisetzen kann, und eine nichtwässrige Elektrolytlösung, wobei die Batterie die nichtwässrige Elektrolytlösung nach einem der Ansprüche 1 bis 6 verwendet.

## Revendications

1. Solution électrolytique non aqueuse pour une utilisation dans une pile secondaire à solution électrolytique non aqueuse comprenant une électrode positive comprenant un matériau actif d'électrode positive capable d'absorber et de libérer un ion métallique et une électrode négative comprenant un matériau actif d'électrode négative capable d'absorber et de libérer un ion métallique, où la solution comprend un composé représenté par la formule (1) suivante, 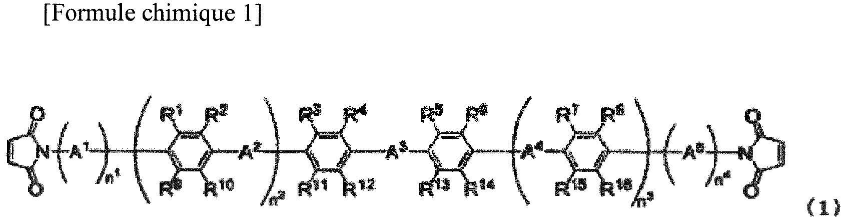
dans laquelle, dans la formule (1), chacun des R¹ à R¹⁶ représente indépendamment l'un quelconque d'un atome d'hydrogène, d'un atome d'halogène, d'un groupe hydrocarboné, d'un groupe représenté par -O-L¹, et d'un groupe représenté par -SO₂-L²,
L¹ et L² représentent un groupe hydrocarboné,
chacun des A¹ à A⁵ représente indépendamment un groupe hydrocarboné divalent, un hétéroatome, ou un groupe ayant un hétéroatome, et
chacun des n¹ à n⁴ représente un nombre entier de 0 ou plus, à condition que quand tous les n¹ à n⁴ sont 0, au moins l'un des R³ à R⁶ et R¹¹ à R¹⁴ représente un groupe autre qu'un atome d'hydrogène, et
dans laquelle le composé représenté par la formule (1) est un composé représenté par la formule (2) suivante, 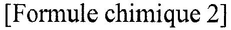 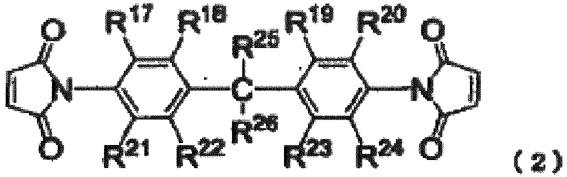
dans laquelle, dans la formule (2), chacun des R¹⁷ à R²⁶ représente indépendamment l'un quelconque d'un atome d'hydrogène, d'un atome d'halogène, d'un groupe hydrocarboné, d'un groupe représenté par -O-L¹, et d'un groupe représenté par -SO₂-L²,
L¹ et L² représentent chacun un groupe hydrocarboné, et
au moins l'un des R¹⁷ à R²⁴ représente un groupe autre qu'un atome d'hydrogène.

2. Solution électrolytique non aqueuse selon la revendication 1, dans laquelle une teneur en composé représenté par la formule (1) est de 0,01 % en poids ou plus et de 5 % en poids ou moins dans la solution électrolytique non aqueuse.

3. Solution électrolytique non aqueuse selon la revendication 1 ou 2, dans laquelle dans le composé représenté par la formule (1), R¹ à R¹⁶ sont un atome d'hydrogène ou un groupe alkyle.

4. Solution électrolytique non aqueuse selon l'une quelconque des revendications 1 à 3, ayant une teneur en eau de 40 ppm en poids ou moins.

5. Solution électrolytique non aqueuse selon l'une quelconque des revendications 1 à 4, comprenant en outre au moins l'un d'un carbonate cyclique comprenant une liaison carbone-carbone insaturée et d'un carbonate cyclique comprenant un atome de fluor.

6. Solution électrolytique non aqueuse selon l'une quelconque des revendications 1 à 5, comprenant en outre un nitrile.

7. Pile secondaire à solution électrolytique non aqueuse comprenant une électrode positive comprenant un matériau actif d'électrode positive capable d'absorber et de libérer un ion métallique, une électrode négative comprenant un matériau actif d'électrode négative capable d'absorber et de libérer un ion métallique, et une solution électrolytique non aqueuse, où la pile utilise la solution électrolytique non aqueuse selon l'une quelconque des revendications 1 à 6.
